# EUROPEAN PATENT SPECIFICATION

(11) **EP 2 039 677 B1**
(45) Date of publication and mention of the grant of the patent: **23.04.2014**
(21) Application number: 07721221.5
(22) Date of filing: 21.05.2007
(51) Int. Cl.: C07C 225/22, C07C 321/24, C07C 391/02, C07D 215/40, C07D 307/91, C08F 4/645, C08F 10/02, C08F 10/04, C08F 2/34

(54) **Organic compounds containing heteroatoms and their use in ziegler-natta catalyst with single activation center**
Heteroatomhaltige organische Verbindungen und ihre Verwendung in Ziegler-Natta-Katalysatoren mit einzelnem Aktivierungszentrum
Composés organiques contenant des hétéroatomes et leur utilisation dans un catalyseur ziegler-natta à centre d'activation unique

(30) Priority: 22.05.2006 CN 200610026766
(43) Date of publication of application: 25.03.2009
(73) Proprietor: SHANGHAI INSTITUTE OF ORGANIC CHEMISTRY, CHINESE ACADEMY OF SCIENCES, Shanghai 200032 (CN)
(72) Inventor: TANG, Yong, Shanghai 200032 (CN); YANG, Xiaohong, Shanghai 200032 (CN); LIU, Bo, Shanghai 200032 (CN); SUN, Xiuli, Shanghai 200032 (CN); MA, Zhi, Shanghai 200032 (CN); GAO, Yuan, Shanghai 200032 (CN); WANG, Cong, Shanghai 200032 (CN)
(74) Representative: Meyer-Dulheuer, Karl-Hermann
(86) International application number: PCT/CN2007/001648
(87) International publication number: WO 2007/134537

(56) References cited:
- EP-A- 1 229 055
- CN-A- 1 884 253
- JP-A- 2001 172 273
- SU-A1- 1 789 524
- SU-A1- 1 819 669
- US-A- 4 759 926
- A.I. URAEV, V.P. KURBATOV ET AL.: "Synthesis and structures of nickel(II) complexes with thioether-containing beta-aminovinyl ketones" RUSSAN CHEMICAL BULLETIN, INTERNATIONAL EDITION, vol. 51, 2002, pages 1924-1927, XP002532477
- MKRTCHYAN E G ET AL: "Synthesis of perfluoroalkyl-containing tri-and tetradentate [beta]-amino enones" RUSSIAN CHEMICAL BULLETIN, KLUWER ACADEMIC PUBLISHERS-PLENUM PUBLISHERS, NE, vol. 54, no. 9, 1 September 2005 (2005-09-01), pages 2150-2156, XP019224816 ISSN: 1573-9171
- KHOSROPOUR A R ET AL: "A mild, efficient and environmentally friendly method for the regio- and chemoselective synthesis of enaminones using Bi(TFA)3 as a reusable catalyst in aqueous media" TETRAHEDRON LETTERS, ELSEVIER, AMSTERDAM, vol. 45, no. 8, 16 February 2004 (2004-02-16), pages 1725-1728, XP004487137 ISSN: 0040-4039
- YAO-CHENG SHI: "3-[(2-Hydroxyphenyl)amino]-1-phenylbut-3- en-1-one ethanol solvate" ACTA CRYSTALLOGRAPHICA SECTION E, vol. E61o, 31 March 2005 (2005-03-31), pages 1130-1132, XP002532478
- D. PAWLICA, M. MARASZALEK, G. MYNARCZUK, L. SIERON AND J. EILMES: "New unsymmetrical Schiff base Ni(II) complexes as scaffolds for dendritic and amino acid superstructures" NEW JOURNAL OF CHEMISTRY, vol. 28, 2004, pages 1615-1621, XP002532479
- ZHANG Z. ET AL.: 'A general and efficient method for the preparation of beta-enamio ketones and esters catalyzed by indium tribromide' ADVANCED SYNTHESIS & CATALYSIS vol. 348, no. 1 + 2, January 2006, pages 184 - 190, XP002431139
- CHEMICAL ABSTRACTS, Columbus, Ohio, US; abstract no. 144:123449, VLASENKO V.G. ET AL.: 'XAFS study of Cu(II), Ni(II) and Co(II) beta-aminovinylketone complexes' & PHYSICA SCRIPTA, T (2005), T115 (12TH X-RAY ABSORPTION FINE STRUCTURE INTERNATIONAL CONFERENCE (XAFS12) 2003, pages 362 - 364
- CHEMICAL ABSTRACTS, Columbus, Ohio, US; abstract no. 138:347859, URAEV A.I. ET AL.: 'Synthesis and structures of nickel(II) complexes with thioether-containing beta-aminovinyl ketones' & RUSSIAN CHEMICAL BULLETIN (TRANSLATION OF IZVESTIYA AKADEMII NAUK, SERIYA KHIMICHESKAYA) vol. 51, no. 10, 2002, pages 1924 - 1927
- KORSHUNOV O.Y. ET AL.: 'Nickel(II) and cobalt(II) chelates with N-naphthyl- and n-quinolyl-beta-aminovinyl ketones' ZHURNAL NEORGANICHESKOI KHIMII vol. 45, no. 9, 2000, pages 1491 - 1497, XP008092047
- QU G. ET AL.: 'Study on internal electron donors in catalyst for propylene polymerization' LIAONING CHEMICAL INDUSTRY vol. 35, no. 1, January 2006, pages 44 - 47, XP008090834
- NEUMAYER D A ET AL: "Approaches to alkaline earth metal-organic chemical vapor deposition precursors. Synthesis and characterization of Barium fluoro-beta-ketoiminate complexes having appended poyether lariats", INORGANIC CHEMISTRY, AMERICAN CHEMICAL SOCIETY, EASTON, US, vol. 37, no. 21, 1 January 1998 (1998-01-01), pages 5625-5633, XP002261922, ISSN: 0020-1669, DOI: 10.1021/IC980208+
- MILTON M D ET AL: "Synthesis of beta-ketoenamine donors having O, N, Se/Te donor functionalities and their reaction chemistry with Pd(II) and Pt(II) metal ions", TETRAHEDRON LETTERS, ELSEVIER, AMSTERDAM, NL, vol. 45, no. 36, 30 August 2004 (2004-08-30), pages 6745-6747, XP004556634, ISSN: 0040-4039, DOI: 10.1016/J.TETLET.2004.07.057
- KATSUMA HIRAKI, TOYOHIKO MASUMOTO, YOSHIO FUCHITA, YASUSHI ZEGI: 'Organopalladium(II) complexes derived fromchloro[4-[2-(diphenylphosphino)ethylami no]-3-penten-2-onato-O,N,P]palladium(II)' BULLETIN OF THE CHEMICAL SOCIETY OF JAPAN vol. 54, 1981, pages 1044 - 1047
- YUN CHI, SUDHIR RANJAN, PO-WEN CHUNG, CHAO-SHIUAN LIU, SHIE-MING PENG, GENE-HSIANG LEE: "Synthesis and characterization of two novel tetranuclear sodiumketoiminate complexes; structural evidence for formation of dative Na....F and Na-C (olefin) bonding interactions", JOURNAL OF THE CHEMICAL SOCIETY, DALTON TRANSACTIONS, 1999, pages 343-347,

## Description

The present invention provides a new class of organic compounds containing heteroatom, their syntheses, and applications as donor in preparing single-site Ziegler-Natta catalysts. Upon activation with alkyl aluminium, alkyl aluminoxane (MAO), or modified alkyl aluminoxane (MMAO), the single-site Ziegler-Natta catalysts can efficiently promote ethylene polymerization or ethylene/α-olefin copolymerization to provide high-performance polyolefin materials.

With the rapid development of polyolefin industry, much more extensive attention have been paid to the production of high-performance polyolefin materials. High-performance polyolefin materials can be prepared mainly in two ways: 1) by excellent single-site catalyst; 2) by advanced technology process. With single-site catalyst (homogeneous catalysts), the properties of polymer could be controlled well and so a variety of high-performance polyolefin materials are provided. However, metal complexes as the real active species of single-site catalysts are unstable, difficult to be synthesized, and difficult in exhibiting their original characters after supported on carrier. All of these difficulties largely limit the applications and development of single-site catalysts. In addition to the aforementioned challenge, a large number of expensive cocatalysts such as alkyl aluminoxane (such as MMAO) is always needed to get high activity.

Compared to the single-site metallocene and non-metallocene catalysts, Ziegler-Natta catalyst are still the most important catalyst now. The main reason is closely related to their stability, simple preparation and low cost. However, because of the character of having multi active sites in Ziegler-Natta catalyst, the polymer structure can not be controlled well when Ziegler-Natta catalyst is used. In recent years, by using advanced Ziegler-Natta catalysts and chemical technology processes, polyolefin materials with excellent performance can be produced. For example: patent US 5,459,116 discloses a kind of olefins polymerization catalyst. The catalyst is prepared by directly reacting a magnesium compound of liquid phase having no reducing power with a titanium compound of liquid phase in the presence of at least one electron donor, which contains at least one hydroxyl group. Superior in activity as well as production yield in polymerizing olefins, the catalyst is capable of not only providing the polymer with high stereoregularity but also improving the bulk density of the polymer, especially polyethylene; U.S. Patent 5,106,807 and 4,330,649 disclose the activity of catalysts and polymer molecular weight can be controlled by the addition of ester compounds; CN1189487C (PCT/KR2000/001549) provides a method to prepare ethylene homopolymers and copolymers with narrow molecular weight distributions 3.6-4.3; Terano reported Ziegler-Natta catalysts supported either on surface functionalized SiO₂ or on ethylene/propylene/diene elastomers (EPDM). The molecular weight distribution of polyethylenes varied from narrow to broad (1.6-30) by solely changing the type of Al-alkyl cocatalyst. This is the narrowest molecular weight distribution obtained by Ziegler-Natta catalyst (Terano, M. Catalysis Commun. 2003, 4, 657-662; Macromol. Chem. Phys. 1998, 199, 1765), however, either the activity of catalyst or the polymer molecular weight decreased significantly.

EP 1229055 A1 provides a process for the (co-) polymerisation of olefins using a Ziegler-Natta type catalyst, in particular to a process for the gas-phase (co-) polymerisation of olefins in a fluidised bed reactor using a Ziegler-Natta type catalyst. Example 6 in D6 discloses a method for the preparation of a Ziegler-Natta type catalyst by suspending silica in hexane at 50°C, adding dibutylmagnesium, followed by adding adding a 2,4-diketone as chelating ligand and subsequently adding TiCl₄, followed by filtration and drying of the solid in vacuum.

The purpose of the invention is to provide a new class of organic compounds containing heteroatoms.

The purpose of the invention is also to provide the application of the organic compounds as electronic donors in the preparation of the single-site Ziegler-Natta catalyst.

The purpose of the invention is also to provide a new class of single-site Ziegler-Natta catalysts and their preparation methods.

The purpose of the invention is also to provide the usage of the catalysts and the catalysts systems. The catalysts and the catalysts systems are highly active to catalyze the ethylene polymerization or copolymerization with α-olefin of C₃-C₁₈, with good control of the polymer molecular weight and well comonomer distribution. The molecular weight distribution (PDI) of the obtained polymer is narrow (PDI 1.6 to 5.0).

The present invention provides a new class of organic compounds containing heteroatoms and their applications as electron donors in the preparation of single-site Ziegler-Natta catalyst, along with magnesium compound and metal compound or/and supporter. The organic compounds may be easily synthesized in high yields under mild conditions by refluxing the corresponding 1, 3-diketone derivatives with amine derivatives in organic solvents for 2-48 hours.

Upon activation with cocatalysts such as alkyl aluminium, the prepared single-site Ziegler-Natta catalysts are highly active for ethylene polymerization or copolymerization with α-olefin of C₃-C₁₈, with the highest activity of ethylene polymerization up to 18000 g polymer/g catalyst; the incorporation ratio of comonomer such as 1-hexene can be higher than 2.0 mol%. The molecular weight distribution of the resulting polymer is narrow (PDI 1.6 to 5.0), and the structure of the polymer is controllable. All of the distinguish characters make the catalyst suitable for commercialization.

The structure of the said organic compounds containing heteroatoms is showed below (I), and in organic solvents which may be a mixture of two tautomerisms I and II:

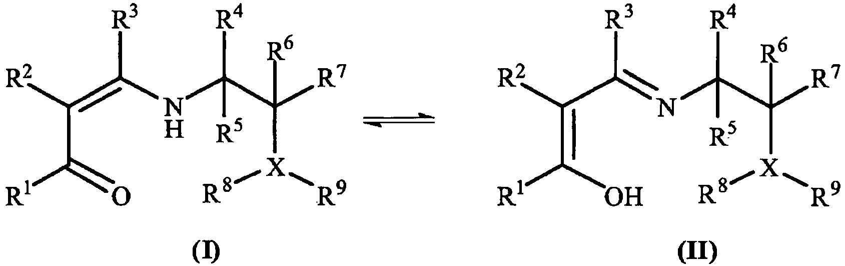

The organic compounds containing heteroatoms provided in the present invention are showed below:

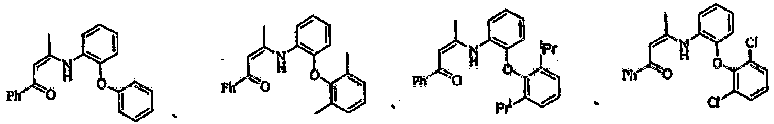

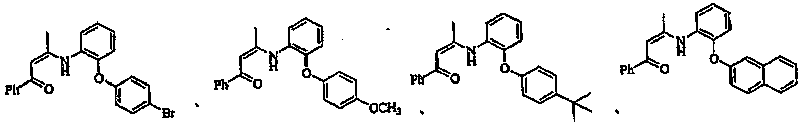

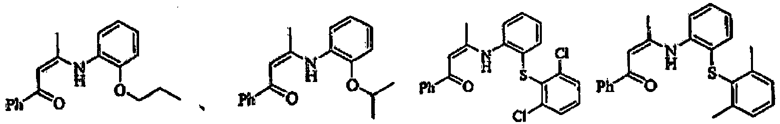

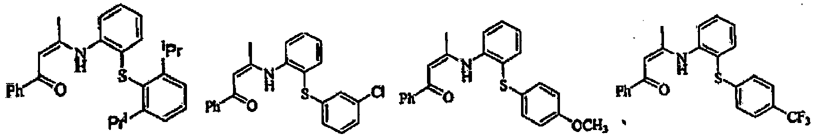

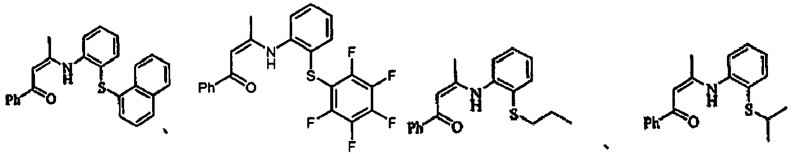

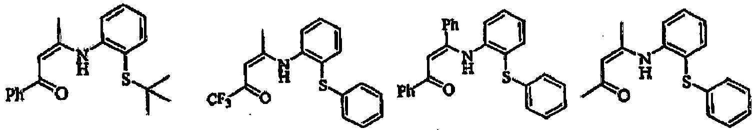

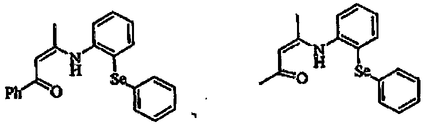

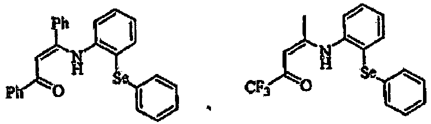

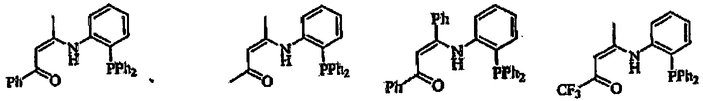

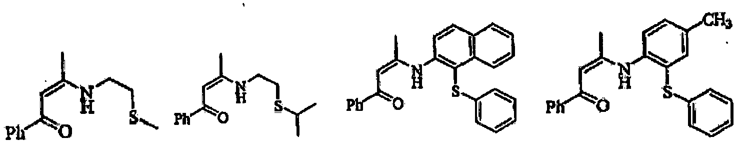

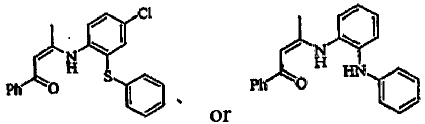

The organic compound containing heteroatom in the invention has a structure of following general formula (I), and which can be a mixture of I and II in organic solvents:

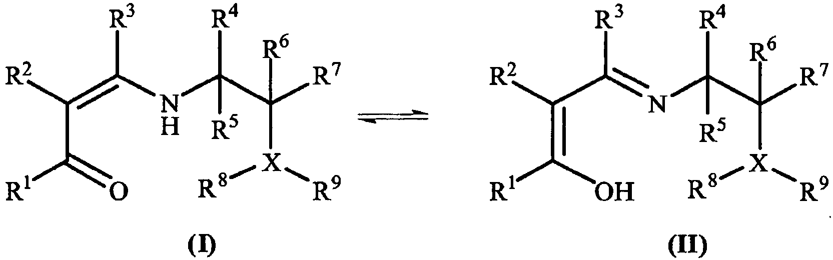

R¹-R⁹ are the groups as aforementioned.

The present invention also relates to a method for preparing a single-site Ziegler-Natta catalyst, wherein the organic compounds containing heteroatoms are used as electron donors, said organic compounds having a formula of 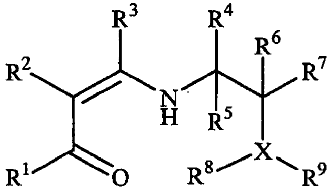 wherein:
R¹ and R² are respectively H, hydrocarbyl of C₁-C₃₀, substituted hydrocarbyl of C₁-C₃₀, aryl group of C₅-C₅₀, or substituted aryl group of C₅-C₅₀, these groups being same or different;
R³, R⁴, R⁵, R⁶ and R⁷ are respectively H, hydrocarbyl group of C₁-C₃₀, substituted hydrocarbyl group of C₁-C₃₀, aryl group of C₅-C₅₀, or substituted aryl group of C₅-C₅₀, these groups being same or different, of which, R⁴, R⁵ with R⁶ or R⁷, R⁶ with R⁸ or R⁹, R⁷ with R⁸ or R⁹ optionally form a bond or a cycle;
R⁸, and R⁹ are respectively hydrocarbyl group of C₁-C₃₀, substituted hydrocarbyl group of C₁-C₃₀, aryl group of C₅-C₅₀, or substituted aryl group of C₅-C₅₀, these groups being same or different, of which, R⁴, R⁵ with R⁶ or R⁷, R⁶ with R⁸ or R⁹, R⁷ with R⁸ or R⁹ optionally form a bond or a cycle;
X is O, S, Se or P; and
when X is O, S or Se, there is only one group, R⁸ or R⁹, on X;
the aryl group is phenyl, naphthyl, anthryl, phenanthryl or other heteroaromatic group;
the substituted hydrocarbyl group or substituted aryl group is the group substituted with hydrocarbyl, halogen, group containing silicon, group containing oxygen atom -OR¹⁰, group containing sulfur atom -SR¹¹ or -S(O)R¹², group containing nitrogen atom-NR¹³R¹⁴ or -N(O)R¹⁵R¹⁶, or group containing phosphorous atom -PR¹⁷R¹⁸ or -P(O)R¹⁹R²⁰;
R¹⁰, R¹¹, R¹², R¹³, R¹⁴, R¹⁵, R¹⁶, R¹⁷, R¹⁸ , R¹⁹, or R²⁰, respectively is substituted hydrocarbyl group of C₁-C₃₀ or aryl group of C₅-C₅₀, these groups being same or different, of which, R¹³ and R¹⁴, R¹⁵ and R¹⁶, R¹⁷ and R¹⁸, R¹⁹ and R²⁰ may link to one another to form covalent bond or to form a ring.

Examples representative of the above mentioned compound I include ED01-ED44, and it needs to emphasize that the compound provided in present invention is not limited to these examples:

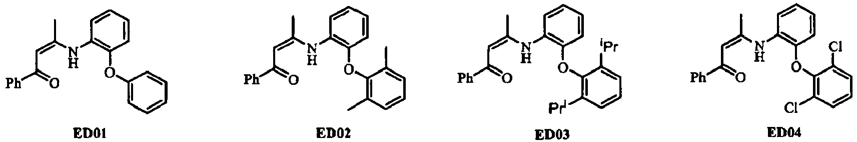

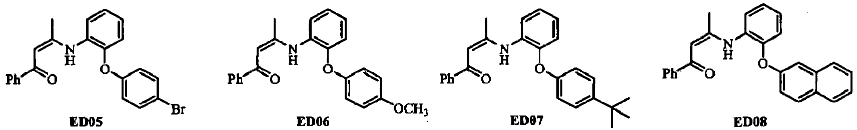

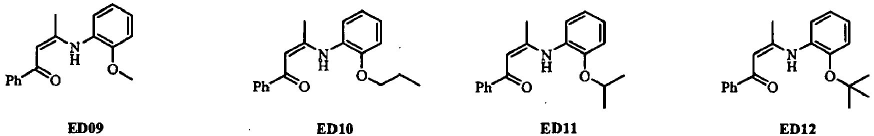

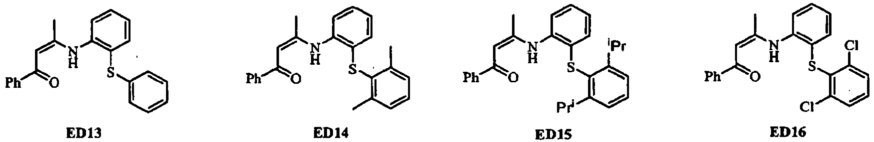

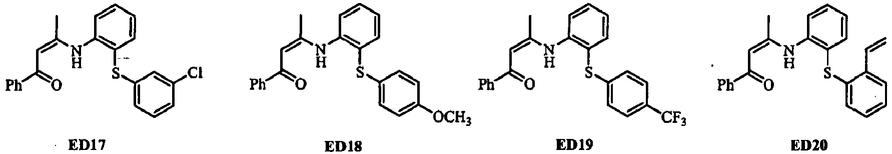

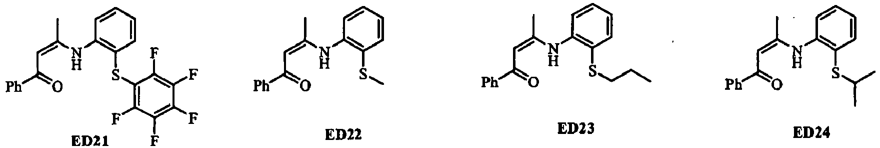

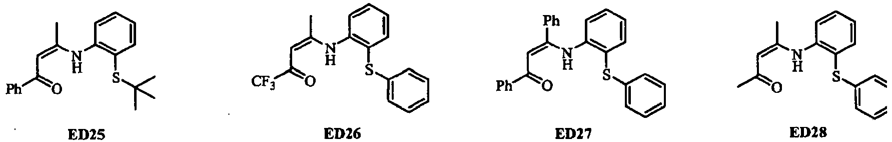

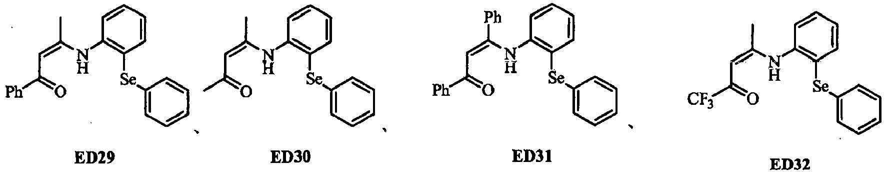

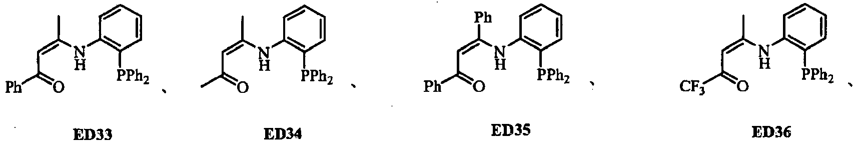

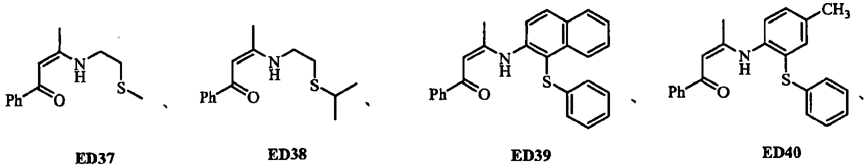

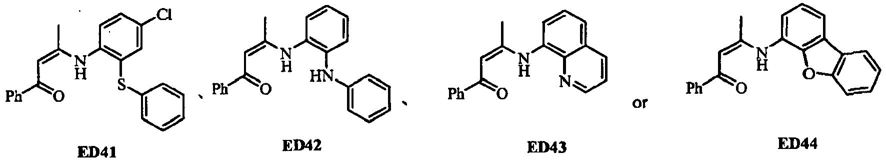

One organic compound or a mixture of two or more of compounds mentioned above can be used as electron donor (ED) in preparing single activation center Ziegler-Natta catalyst, preferably ED01, ED02, ED06-ED11, ED13-ED14, ED18, ED21-ED28, ED33-36, and ED37-ED44; more preferably, ED01, ED06, ED09, ED13, ED14, ED18, ED21-ED24, ED27-ED28, ED33, ED35, ED37-ED38.

The organic compounds containing heteroatoms can be prepared according to literature methods (Hu W.-Q. et. al., Organometallics 2004, 23, 1684-1688; Wang, C. et. al. Macromol. Rapid Commun. 2005, 26, 1609-1614).

In the invention, the compound is prepared in organic solvent by mixing diketone derivative(**III**) with amine derivative (**IV**) in the presence of catalyst. The mixture is refluxed for 2-48 hrs, and after removing the solvent, the residue is purified by recrystallization in alcohol solvent to get compound (**I**).

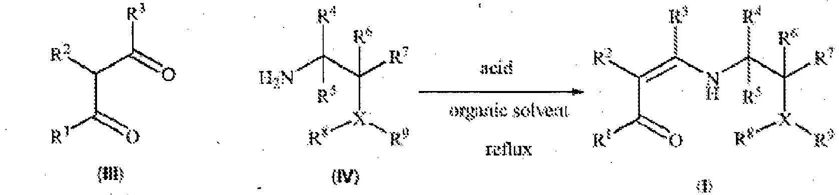

The catalyst in the reaction is preferably formic acid, acetic acid, TsOH, or the other organic acid, the organic solvent is methanol, ethanol, or anhydrous ethanol, preferably anhydrous ethanol;
the mole ratio of diketone to amine to catalyst is 1-1.5: 1: 0.01-0.1;
diketone is described by formula(**III**): 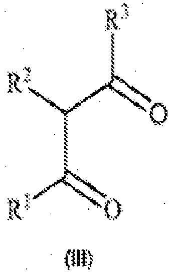 amine can be described by formula (**IV**): 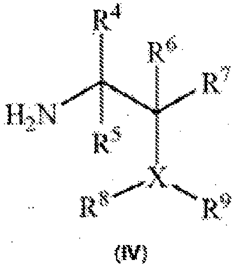 R¹-R⁹ are the groups as those mentioned above.

In the invention, the single activation center Ziegler-Natta catalyst is made of magnesium compound, carrier, metallic compound and the organic compound containing heteroatoms, and the content of metal is in the range of 0.1-15 wt%.

The magnesium compound can be magnesium halide, alkyl magnesium, alkoxy magnesium halide, alkoxy magnesium or magnesium halide coordinate alcohol. Of the above named magnesium compound, a mixture of two or more may also be used; the magnesium halide or alkyl magnesium is more preferable.

The carrier of the single activation center Ziegler-Natta catalyst is an organic material or inorganic oxides containing the metal oxides of the group 2, 4, 13 and 14. The inorganic oxides is Al₂O₃, SiO₂, a mixture of oxides, clay, molecular sieve, or oxide materials provided by a gaseous metal oxide or silica compound through hydrolysis at high temperature;

The "metallic compound" can be represented by the formula (**V**):

MLₐ (**V**)

wherein:
a is 3,4, 5 or 6;
L is selected from halogen atom, hydrocarbyl group of C₁-C₃₀, group containing oxygen atom, group containing nitrogen atom; each L in the formula may be same or different, and they may link to one another to form a bond or form a ring;
the halogen atom is F, Cl, Br, or I;
the substituted hydrocarbyl group is the group substituted with hydrocarbyl, halogen, carbonyl group, ester group, group containing silicon, group containing oxygen atom, group containing sulfur atom, group containing nitrogen atom, group containing phosphorous atom, or group containing selenium atom;
the group containing oxygen atom is selected from alkoxy -OR²³, tetrahydrofuran or diethyl ether; the group containing nitrogen atom is selected from - NR²⁴R²⁵ or - N(O)R²⁶R²⁷;
R²³-R²⁷ respectively is H, hydrocarbyl group of C₁-C₃₀ or aryl group of C₅-C₅₀; these groups may be same or different, and R²⁴ with R²⁵, R²⁶ with R²⁷ may be independent, form a bond or to form a ring;
the absolute value of the total negative charge of L in the formula (**V**) is the same as the absolute value of the positive charge of metal M, so as to ensure the electroneutrality of the metal compound.
M is a transition metal atom of group 4 to group 6, preferably titanium, zirconium, chromium or vanadium.

Examples of the "metallic compound" include titanium compound, zirconium compound, chromium compound, or vanadium compound.

Titanium compound may be tetrahalogenated titanium or tetrahalogenated titanium coordinated with THF or Et₂O, preferably TiCl₄, TiCl₄(THF)₂; or alkoxy trihalogenated titanium, preferably Ti(OCH₃)Cl₃, Ti(OC₂H₅)Cl₃ or Ti(OC₂H₅)Br₃; or alkoxy dihalogenated titanium, preferably Ti(OCH₃)₂Cl₂, Ti(OC₂H₅)₂Cl₂; or alkoxy halogenated titanium, preferably Ti(OCH₃)₃Cl, Ti(OC₂H₅)₃Cl; or tetraalkoxy titanium, tetraamido titanium or tetraalkyl titanium.

Zirconium compound prefers ZrCl₄ or tetraamido zirconium.

Chromium compound prefers CrCl₃ or CrCl₃(THF)₃.

Vanadium compound prefers VCl₅, VCl₃(THF)₃ or VCl₃(PMe)₃.

More preferable "metallic compound" is TiCl₄, TiCl₄(THF)₂, Ti(NMe₂)₄, Ti(NEt₂)₄, Ti(CH₂Ph)₄, ZrCl₄, Zr(NMe₂)₄, Zr(NEt₂)₄, CrCl₃, CrCl₃(THF)₃, VCl₃, or VCl₃(THF)₃.

The most preferable "metallic compound" is TiCl₄, TiCl4(THF)₂, Ti(CH₂Ph)₄, ZrCl₄, CrCl₃, CrCl₃(THF)₃ or VCl₃(THF)₃.

Preparation of the single activation center Ziegler-Natta catalyst

In the invention, the organic compound containing heteroatoms is used effectively as an electron donor (ED) to prepare a single activation center Ziegler-Natta catalyst by the following procedure:
(1) pretreating a carrier of an organic or an inorganic solid or a mixture of them by heating;
(2) dissolving magnesium compound in THF to form a solution at room temperature to 70°C;
(3) to the aforesaid solution (2) was added the pretreated solid (carrier), metallic compound of (1) and the electron donor, the resulting mixture was kept for several hours under certain temperature, and then removing the solvent, and the residue was washed with inert hydrocarbon solvent and was dried under reduced pressure to provide single activation center Ziegler-Natta catalyst.

In step (1), the solid, which is used as a carrier, is treated at 30-1000°C for 1-24 hrs under inert atmosphere or reduced pressure; and the optimal carrier is silica with particle size of 1-50 µm, specific surface area of 100-300 m²/g, pore volume of 0.5-3 mL/g, and an average pore diameter of 10-50 nm.

In step (2), the ratio of magnesium compound to THF is 1g : 1-100 mL, preferably 1g : 20-80 mL.

In step (3), the weight ratio of magnesium compound to carrier is 1 : 0.1-20, preferably 1 : 0.5-10; the mole ratio of magnesium compound to metallic compound is 0.5-100 : 1, preferably 0.5-50 : 1; the mole ratio of electron donor (ED) to metallic compound is 0.01-10 : 1, preferably 0.1-5 : 1; the reaction temperature is room temperature to 100 °C, preferably 50 - 70 °C; reaction time is 2-48 hrs, preferably 4-24 hrs.

In step (3), the inert hydrocarbon solvent is hydrocarbon of C₅-C₁₀ or arene of C₆-C₈, which is selected from pentane, hexane, decane, heptane, octane or toluene, preferably hexane or toluene.

In step (3), it is workable to treat magnesium compound with metallic compound for 2-48 hrs at room temperature to 100 °C first, then with the pretreated carrier, and finally with electron donor for 2-48 hrs at room temperature to 100 °C. After removing the solvent, the residue was washed with inert hydrocarbon solvent and dried to provide Ziegler-Natta catalyst; the procedure can also be carried out by the following sequence: treating magnesium compound with a carrier for 2-48 hrs at room temperature to 100 °C to get a composite carrier which then react with a solution of an electronic donor and metallic compound for 2-48 hrs at room temperature to 100 °C, and by the same treatment mentioned above to provide the desired catalyst.

In the invention, the solvents used during preparing single activation center Ziegler-Natta catalyst are treated to remove water and oxygen strictly and all manipulations were performed under inert atmosphere using standard Schlenk techniques which will not be described again in the following examples.

The catalyst in this invention is suitable for ethene homo-polymerization, ethene/α-olefin combined polymerization and ethene/cycloolefin combined polymerization. Alkyl aluminium, alkyl aluminoxane or a mixture of two or more of them is used as catalyst promoter in the polymerization process. A suitable catalyst promoter is selected from AlEt₃, Al(i-Bu)₃, AlEt₂Cl, Al(n-Hex)₃, MAO, EAO, MMAO or a mixture of two or more of them, preferably AlEt₃, MMAO or a mixture of them; the suitable mole ratio of Al/Ti is 20-1000, preferably 20-500; the useful α-olefins in the invention are C₃-C₂₀ such as propene, 1-butene, 1-hexene, 1-octene, 1-heptene, 4-methyl-1-petene, 1-decene, 1-undecene, 1-dodecene, 1-tetradecene, 1-hexadecene, 1-octadecene; the cycloolefins are cyclopetene, cyclohexene, norbornene or alkyl substitute thereof. Either α-olefins or cycloolefins used in the present polymerization optionally contain hydroxyl group, carboxyl group, ester group, or amine group.

The polymerization can be run in slurry process or gas process.

In the case of slurry polymerization process, the polymerization is generally performed at 80-120 °C under a total pressure of 0.1-10 MPa with 0-1.0 MPa hydrogen pressure; the polymerization may be carried out under supercritical or subcritical state with inert solvent such as propane, isobutane or hexane as solvent; either autoclave or loop reactor are useful.

In the case of gas polymerization process, the polymerization is generally conducted under a total pressure of 0.1-10 MPa at 40-100 °C in gas fluidized bed or gas autoclave.

The metal mass content of the prepared single activation center Ziegle -Natta catalyst is measured by ICP-AES, OPTRMA-3000 inductively coupled plasma atomic emission spectrometry.

Molecular weight and molecular weight distribution of the polymers are measured by Waters Alliance GPC2000 (differential refractive index detector) at 135 °C and 1,2,4-trichlorobenzene as eluent (flow rate 1.0 mL/min) polystyrene as a reference sample.

The ¹³C NMR of the polymer was measured by Varian XL-300 MHz nuclear magnetic resonance spectrometer at 110 °C in D₄-o-dichlorobenzene. And the incorporation ratio of the comonomer is calculated by the literature method (J. C. Randall, JMS-Rev. Maromol. Chem. Phys. 1989, C29(2&3), 201-317.).

Figure **1** is an X-ray of compound ED14 in example **9**.

Figure **2** is a ¹³C NMR of the ethene/1-hexene copolymer in example 78.

### Example 1. Synthesis of electron donor (ED):

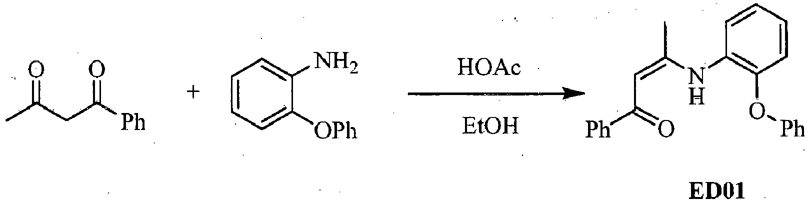

To a solution of 1-phenyl-1,3-butanedione (42.0 mmol) and 2-phenoxybenzenamine (40.0 mmol) in anhydrous ethanol (30 mL) was added acetic acid (3 mL). After refluxing for 30 hrs, the resulting mixture was cooled naturally and the solvent removed under reduced pressure,filtered to give a yellow solid which is recrystallized from anhydrous ethanol to give ED01. Yield 5.534 g (42%). ED01: ¹H NMR (300 MHz, CDCl3): δ (ppm) 12.82 (s, 1 H), 7.87-7.84 (m, 2H), 7.44-6.91 (m, 12H), 5.86 (s, 1H), 2.12 (s, 3H); ¹³C NMR (75 MHz, CDCl3): δ (ppm) 188.83, 162.14, 155.35, 150.68, 139.91, 130.85, 130.29, 129.68, 128.52, 128.18, 127.06,127.01, 126.87, 124.03, 119.77, 119.53, 94.65, 20.34.

### Example 2-7

Examples **2-7** provide some examples of the prepared electron donor (ED):

The same procedure as that for the preparation of ED01 was used. These compounds were prepared with the corresponding diketone derivatives and amine derivatives. The characteristic data of the ED are shown as following.

### Example 2: ED02: ¹H NMR (300 MHz, CDCl3): δ (ppm) 13.02 (s, 1 H),

7.94-7.91 (m, 2H), 7.44-6.98 (m, 9H), 6.45-6.42 (m, 1 H), 5.96 (s,1 H), 2.34 (s,3H), 2.15 (s, 6H); ¹³C NMR (75 MHz, CDCl3): δ (ppm) 188.76, 162.72, 151.32, 151.06, 140.11, 131.22, 130.73, 128.99, 128.16, 127.36, 127.09, 126.91, 126.44, 125.25, 121.29, 113.45, 94.43, 20.48, 16.32.

Example **3**: ED05: ¹H NMR (300 MHz, CDCl3): δ (ppm) 12.82 (s, 1 H), 7.88-7.85 (m, 2H), 7.44-6.86 (m, 11 H), 5.87 (s, 1 H), 2.13 (s, 3H); ¹³C NMR (75 MHz, CDCl3): δ (ppm) 188.86, 162.13, 155.96, 150.53, 139.91, 132.63, 130.85, 130.38, 128.19, 127.07, 127.02, 126.89, 124.13, 120.13, 119.69, 115.97, 94.68, 20.32.

Example **4**: ED06: ¹H NMR (300 MHz, CDCl3): δ 12.82 (s, 1 H), 7.88-7.84 (m, 2H), 7.42-7.38 (m, 5H), 7.10-6.84 (m, 6H), 5.86 (s, 1 H), 3.72 (s, 3H), 2.09 (s, 3H).

Example **5**: ED07: ¹H NMR (300 MHz, CDCl3): δ (ppm) 12.84 (s, 1 H), 7.89-7.86 (m, 2H), 7.43-6.93 (m, 11 H), 5.87 (s, 1 H), 2.15 (s, 3H), 1.28 (s, 9H).

Example **6**: ED08: ¹H NMR (300 MHz, CDCl3): δ (ppm) 12.91 (s, 1 H), 7.85-7.79 (m, 5H), 7.42-7.26 (m, 11H), 5.85 (s,1H), 2.17 (s, 3H); ¹³C NMR (75 MHz, CDCl3): δ (ppm) 188.72, 162.35, 154.31, 151.14, 139.97, 134.14, 130.73, 130.29, 130.15, 129.91, 128.12, 127.69, 127.09, 127.04, 126.96, 126.82, 126.47, 124.77, 123.63, 119.78, 119.41, 114.20, 94.58, 20.37.

Example **7**: ED09: ¹H NMR (300 MHz, CDCl3): δ 12.82 (s, 1 H), 7.95-7.91 (m, 2H), 7.41-7.14 (m, 7H), 5.92 (s, 1 H), 3.64 (s, 3H), 2.06 (s, 3H).

### Example 8

To a solution of 1-phenyl-1,3-butanedione (10.0 mmol) in CH₃OH (15 mL) was added 2-(phenylthio)benzenamine (10.0 mmol), and then was added formic acid (0.5 mL). After refluxing for 48 hrs, the resulting mixture was cooled naturally and the solvent removed under reduced pressure,filtered to give a yellow solid which was recrystallized from ethanol and dried to give ED13. Yield 1.8156 g (53%). ¹H NMR (300 MHz, CDCl3): δ (ppm) 12.93 (s, 1H), 7.94-7.91 (m, 2H), 7.47-7.38 (m, 5H), 7.31-7.15 (m, 7H), 5.85 (s, 1H), 1.97 (s, 3H); ¹³C NMR (75 MHz, CDCl3): δ (ppm) δ 188.84, 162.06, 139.94, 137.78, 133.95, 133.52, 132.55, 131.20, 130.83, 129.25, 127.78, 127.22, 127.14, 126.99, 126.88, 94.32, 20.06,. IR: 3060, 1597, 1574, 1546, 1508, 1462, 1425, 1317, 1287, 1271, 1060, 760, 747, 732cm⁻¹; LRMS-EI(m/z): 345 (M⁺), 91 (100); elemental analysis for C₂₂H₁₉NOS: calculated value: C, 76.49; H, 5.54; N, 4.05; measured value: C, 76.64; H, 5.63; N, 3.77.

### Example 9

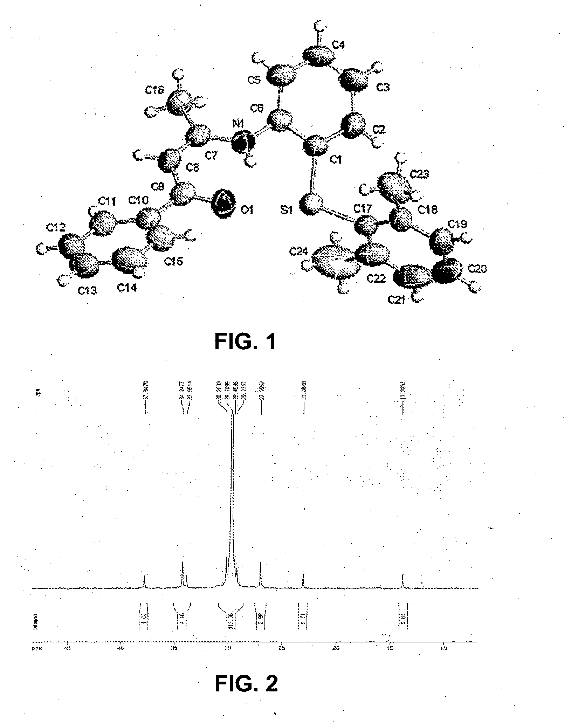

To a solution of 1-phenyl-1,3-butanedione (1.92 mmol) in anhydrous C₂H₅OH (7 mL) was added 2-(2,6-dimethylphenylthio)benzenamine (1.74 mmol), and then was added acetic acid (0.6 mL). After refluxing for 24 hrs, the resulting mixture was cooled naturally and the solvent removed under reduced pressure, filtered to give a yellow solid which was recrystallized from ethanol and dried to give ED14. Yield 0.4657 g (72%) ¹H NMR (300 MHz, CDCl3): δ 12.84 (s, 1H), 7.99-7.96 (m, 2H), 7.46-7.43 (m, 3H), 7.25-7.01 (m, 6H), 6.46-6.43 (m, 1 H), 5.99 (s, 1 H), 2.40 (s, 6H), 2.06 (s, 3H); ¹³C NMR (75 MHz, CDCl3): δ (ppm) 188.93, 163.21, 144.06, 139.91, 136.21, 135.14, 130.83, 129.45,129.17, 128.54, 128.15, 127.50, 127.15, 124.86, 124.76, 93.95, 21.68, 20.01; IR: 3450, 3060, 2920, 1599, 1577, 1550, 1461, 1317, 1284, 747cm⁻¹; LRMS-EI(m/z): 373 (M⁺), 105 (100); elemental analysis for C₂₄H₂₃NOS: calculated value: C, 77.18; H, 6.21; N, 3.75; measured value: C, 77.44; H, 6.18; N, 3.34. Molecular structure of ED14 is shown in Figure **1**.

### Example 10

To a solution of 1-phenyl-1,3-butanedione (1.16 mmol) in anhydrous C₂H₅OH (7 mL) was added 2-(2,6-diisopropylphenylthio)benzenamine (1.05 mmol), and then was added formic acid (0.2 mL). After refluxing for 8 hrs, the resulting mixture was cooled naturally and the solvent removed under reduced pressure,filtered to give a yellow solid which was recrystallized from ethanol and dried to give ED15 (0.3203 g, 71%): ¹H NMR (300 MHz, CDCl3): δ 12.82 (s, 1H), 8.00-7.97 (m, 2H), 7.48-7.01 (m, 9H), 6.40-6.37 (m, 2H), 6.02 (s, 1 H), 2.08 (s, 3H), 1.15-1.12 (d, J= 7.2Hz, 12H); ¹³C NMR (75 MHz, CDCl3): δ (ppm) 188.98, 154.22, 139.96, 138.55, 130.87, 130.45, 128.22, 127.57, 127.50, 127.20, 126.78, 125.34, 124.55, 124.24, 93.89, 31.66, 24.17, 19.96; IR: 3060, 2960, 1597,1557, 1461, 1319, 1284, 745cm⁻¹; LRMS-EL (m/z): 430 (M⁺), 252 (100); elemental analysis for C₂₈H₃₁NOS: calculated value: C, 78.28; H, 7.27; N, 3.26; measured value: C, 78.29; H, 7.51; N, 3.07.

### Example 11

To a solution of 1-phenyl-1,3-butanedione (1.22 mmol) in anhydrous C₂H₅OH (10 mL) was added 2-(2,6-dichlorophenylthio)benzenamine (1.11 mmol), and then was added formic acid (0.5 mL). After refluxing for 20 hrs, the resulting mixture was cooled naturally and the solvent removed under reduced pressure, filtered to give a yellow solid which was recrystallized from ethanol and dried to give ED16 (0.3363 g, 73%): ¹H NMR (300 MHz, CDCl3): δ 12.81 (s, 1H), 7.97-7.94 (m, 2H), 7.47-7.10 (m, 8H), 6.73-6.70 (m, 1 H), 5.96 (s, 1 H), 2.06 (s, 3H); ¹³C NMR (75 MHz, CDCl3): δ (ppm) 189.00, 163.02, 141.70, 139.87, 136.02, 134.02, 130.86, 130.83, 130.29, 128.95, 128.17, 127.84, 127.57, 127.18, 126.83, 126.22, 94.16, 20.08; IR: 3420, 3060, 1600, 1578, 1553, 1426, 1317, 1283, 782, 750cm⁻¹; LRMS-EI (m/z): 414 (M⁺), 105 (100); elemental analysis for C₂₂H₁₇Cl₂NOS: calculated value: C, 63.77; H, 4.14; N, 3.38; measured value: C, 63.54; H, 4.04; N, 3.20.

### Example 12-15

ED17-ED22 were prepared from the corresponding diketone derivatives and amine following the procedures of example **11**:

Example **12**: ED17: ¹H NMR (300 MHz, CDCl₃): δ 12.95 (s, 1 H), 7.91-7.88 (m, 2H), 7.47-7.15 (m, 11 H), 5.81 (s, 1 H), 1.95 (s, 3H); ¹³C NMR (75 MHz, CDCl3): δ (ppm) 189.00, 161.53, 139.83, 138.97, 136.57, 134.73, 132.99, 131.35, 130.89, 130.69, 130.08, 129.32, 128.44, 128.15, 127.39, 127.12, 126.99, 126.83, 94.59, 19.99.

Example **13**: ED18: ¹H NMR (300 MHz, CDCl₃): δ 12.89 (s, 1 H), 7.97-7.94 (m, 2H), 7.44-7.40 (m, 5H), 7.13-6.86 (m, 6H), 5.91 (s, 1 H), 3.80 (s, 3H), 1.99 (s, 3H); 13C NMR (75 MHz, CDCl3): δ (ppm) 188.83, 162.59, 160.10, 139.92, 136.71, 136.21, 136.02, 130.85, 128.51, 128.18, 127.17, 127.03, 125.96, 122.36, 115.06, 109.71, 94.10, 55.32, 20.12.

Example **14**: ED21: ¹H NMR (300 MHz, CDCl3): δ 12.86 (s, 1H), 7.95-7.92 (m, 2H), 7.48-7.20 (m, 7H), 5.95 (s, 1H), 2.00 (s, 3H); ¹³C NMR (75 MHz, CDCl3): δ (ppm) 189.28, 162.20, 139.60, 138.08, 131.07, 130.53, 128.42, 128.25, 128.06, 127.61, 127.15, 94.40, 19.96.

Example 15: ED22: 1 H NMR (300 MHz, CDCl3): δ 12.82 (s, 1 H), 7.94-7.90 (m, 2H), 7.41-7.13 (m, 7H), 5.91 (s, 1 H), 2.47 (s, 3H), 2.04 (s, 3H)

### Example 16

To a solution of benzoyl acetone (5.54 mmol) in anhydrous C₂H₅OH (5 mL) was added 1-(phenylthio)propan-2-amine (5.54 mmol), and then was added formic acid (0.5 mL). After refluxing for 36 hrs, the resulting mixture was cooled naturally and the solvent removed under reduced pressure,filtered to give a yellow solid which was recrystallized from ethanol and dried to give ED23 (1.7245 g, 68%). ¹H NMR (300 MHz, CDCl3): δ 12.86 (s, 1H), 7.96-7.94 (m, 2H), 7.46-7.17 (m, 7H), 5.94 (s, 1 H), 2.89-2.84 (t, J=7.2Hz, 2H), 2.04 (s, 3H), 1.71-1.64 (m, 2H), 1.05-1.00 (t, J=7.5Hz, 3H). ¹³C NMR (75 MHz, CDCl3): 188.83, 162.45, 139.97, 137.53, 134.23, 130.83, 128.83, 128.17, 127.19, 126.85, 126.75, 125.89, 94.21, 34.64, 22.28, 20.23, 13.52; IR: 3060, 2962, 1598, 1574, 1548, 1515, 1461, 1432, 1317, 1280, 1195, 1064, 754, 708cm⁻¹; LRMS-EI(m/z): 311(M⁺), 105 (100); elemental analysis for C₁₉H₂₁NOS: calculated value: C, 73.27; H, 6.80; N, 4.50; measured value: C, 73.20; H, 6.81; N, 4.23.

### Example 17-20

ED24-ED27 were prepared from the corresponding diketone derivatives and amine following the procedures of example **16**:

Example **17**: ED24: ¹H NMR (300 MHz, CDCl3): δ 12.94 (s, 1H), 7.97-7.93 (m, 2H), 7.49-7.17 (m, 7H), 5.93 (s, 1 H), 3.41-3.37 (m, 1 H), 2.07 (s, 3H), 1.31-1.29 (d, J=6Hz, 6H); ¹³C NMR (75 MHz, CDCl3): δ (ppm) 188.67, 161.81, 139.95, 139.17, 132.57, 131.96, 130.75, 128.11, 127.10, 126.34, 126.20, 94.43, 37.50, 22.91, 20.34; IR: 3060, 2980, 1598, 1577, 1511, 1436, 1320, 1280, 758, 703, 673 cm⁻¹; LRMS-EI(m/z): 311 (M⁺), 105 (100); elemental analysis for C₁₉H₂₁NOS: calculated value: C, 73.27; H, 6.80; N, 4.50; measured value: C, 73.19; H, 6.74; N, 4.14.

Example **18**: ED25: ¹H NMR (300 MHz, CDCl3): δ (ppm) 13.17 (s, 1 H), 7.98-7.94 (m, 2H), 7.66-7.63 (m, 1 H), 7.47-7.17 (m, 6H), 5.93 (s, 1 H), 2.14 (s, 3H), 1.32 (s, 9H); 13C NMR (75 MHz, CDCl3): δ (ppm) 188.53, 160.52, 143.05, 139.82, 130.80, 129.64, 128.16, 127.20, 125.20, 125.02, 95.17, 47.86, 30.84, 20.82; IR: 3060, 2980, 1596, 1577, 1555, 1456, 1321, 1280, 759 cm⁻¹; LRMS-EI(m/z): 325 (M⁺), 105 (100); elemental analysis for C₂₀H₂₃NOS: calculated value: C, 73.81; H, 7.12; N, 4.30; measured value: C, 73.73; H, 7.07; N, 3.95.

Example **19**: ED26: ¹H NMR (300 MHz, CDCl3): δ (ppm) 12.35 (s, 1 H), 7.34-7.17 (m, 8H), 5.44 (s, 1H), 1.91 (s, 3H); ¹³C NMR (75 MHz, CDCl3): δ (ppm) 176.53, 167.93, 136.39, 134.25, 132.97, 132.34, 131.83, 129.41, 128.30, 128.09, 127.70, 127.25, 115.47, 90.81 (t), 19.92; IR: 3155, 2925, 2852, 1620, 1590, 1565, 1467, 1439, 1428, 1292, 1241, 1062, 861, 753, 745, 734cm⁻¹; elemental analysis for C₁₇H₁₄F₃NOS: calculated value: C, 60.52; H, 4.18; N, 4.15; measured value: C, 60.68; H, 4.15; N, 3.95.

Example **20**: ED27: ¹H NMR (300 MHz, CDCl₃): δ (ppm) 12.91 (s, 1 H), 7.99-6.41 (m, 18H), 6.08 (s, 1H); ¹³C NMR (75 MHz, CDCl3): δ (ppm) 189.59, 160.50, 139.94, 139.68, 135.87, 134.50, 132.95, 131.91, 131.31, 129.61, 129.12, 128.91, 128.44, 128.27, 128.03, 127.53, 127.41, 127.36, 124.90, 124.46, 97.92; IR: 3051, 1545, 1480, 1438, 1330, 1282, 1207, 1050, 1022, 781, 754, 686cm⁻¹; elemental analysis for C₂₇H₂₁NOS: calculated value: C, 79.57; H, 5.19; N, 3.44; measured value: C, 79.23; H, 5.18; N, 3.13.

### Example 21

To a solution of acetylacetone (10 mmol) in CH₃OH (15 mL) was added 2-(phenylthio)benzenamine (10 mmol), and then was added formic acid (1 mL). After refluxing for 24 hrs, solvent was removed and the residue was cooled. The generated solid was collected and recrystallized from ethanol and dried to give ED28 (1.8156 g, 52.6%). ¹H NMR (300 MHz, CDCl3): δ (ppm) 12.34 (s, 1 H), 7.35-7.26 (m, 5H), 7.19-7.11 (m, 4H), 5.15 (s, 1 H), 2.09 (s, 3H), 1.84 (s,3H); ¹³C NMR (75 MHz, CDCl3): δ (ppm) 196.30, 159.95, 137.83, 133.68, 132.36, 131.14, 129.20, 128.92, 127.71, 127.17, 126.85, 126.66, 126.33, 97.77,29.15,19.49. IR: 3058, 1575, 1500, 1462, 1439, 1377, 1355, 1275, 1186, 1063, 1024, 993, 921, 751, 691, 660cm⁻¹; LRMS-EI(m/z): 283 (M⁺), 174 (100); elemental analysis for C₁₇H₁₇NOS: calculated value: C, 72.05; H, 6.05; N, 4.94; measured value: C, 72.09; H, 6.02; N, 4.78.

### Example 22-31

ED33-44 were prepared following the procedure shown in example **21**.

Example **22**: ED33: ¹H NMR (300 MHz, CDCl3): δ (ppm) 12.90 (s, 1 H), 7.93-7.14 (m, 19H), 5.81 (s, 1 H), 1.94 (s, 3H).

Example **23:** ED35: ¹H NMR (300 MHz, CDCl3): δ (ppm) 12.87 (s, 1 H), 7.95-7.11 (m, 24H), 6.35 (s, 1 H).

Example **24**: ED37: ¹H NMR (300 MHz, CDCl3): δ 13.08 (s, 1H), 7.81-7.49 (m, 5H), 5.77 (s, 1 H), 3.02 (t, 2H), 2.70 (t, 2H), 2.09 (s, 3H), 1.96 (t, 3H).

Example **25**: ED38: ¹H NMR (300 MHz, CDCl3): δ 13.28 (s, 1H), 7.81-7.49 (m, 5H), 5.77 (s, 1 H), 3.02 (t, 2H), 2.88 (m, 1 H), 2.70 (t, 2H), 1.95 (t, 3H), 1.25(d, 6H).

Example **26:** ED39: ¹H NMR (300 MHz, CDCl3): δ 12.38 (s, 1 H), 7.81-6.56 (m, 16H), 5.99 (s, 1 H), 1.71 (t, 3H).

Example **27**: ED40: ¹H NMR (300 MHz, CDCl3): δ 12.38 (s, 1H), 7.81-7.49 (m, 5H), 7.44-7.22 (m, 5H), 6.75-6.14 (m, 3H), 5.99 (s, 1 H), 2.35 (s, 3H), 1.71 (s, 3H).

Example **28:** ED41: ¹H NMR (300 MHz, CDCl3): δ 12.89 (s, 1 H), 7.97-7.64 (m, 5H), 7.44-7.22 (m, 5H), 6.66-6.24 (m, 3H), 5.95 (s, 1 H), 1.91 (s, 3H).

Example **29**: ED42: ¹H NMR (300 MHz, CDCl3): δ 12.38 (s, 1H), 9.77 (s, 1 H), 7.81-7.49 (m, 5H), 7.33-6.98 (m, 5H), 6.61-6.21 (m, 4H), 5.99 (s, 1 H), 1.71 (s, 3H).

Example **30:** ED43: ¹H NMR (300 MHz, CDCl3): δ 12.38 (s, 1H), 8.80 (s, 1 H), 7.94-6.84 (m, 10H), 5.97 (s, 1 H), 1.73 (s, 3H).

Example **31**: ED44: ¹H NMR (300 MHz, CDCl3): δ 12.40 (s, 1H), 7.98-6.39 (m, 12H), 5.95 (s, 1 H), 1.75 (s, 3H).

### Example 32

The preparation of single activation center Ziegler-Natta catalyst of the invention:
(1) thermo-pretreatment of the carrier
   ES70 silica (product of Ineos company) is calcinated under nitrogen atmosphere at 200 °C for 2 hrs and then for 4 hrs at 400 °C, after that it is cooled under nitrogen atmosphere to provide carrier ES70.
(2) the preparation of single activation center Ziegle-Natta catalyst Methodone:
   Anhydrous MgCl₂ (1.0 g) was added to tetrahydrofuran (THF 40 mL), stirred at 60 °C for 2 hrs; then to the solution was added TiCl₄ (3.4 mmol) and the reaction mixture was heated at 60 °C for 4 hrs. Then the pretreated ES70 carrier (1.0 g) was added and the resulting mixture was heated for further 4 hrs at 60 °C. To the mixture was added desired electron donor (4.0 mmol) and the reaction system was maintained at 60 °C for another 12 hrs. Then, the solvent was removed under reduced atmosphere, and the residue was washed with hexane (3x20 mL) and then dried under vacuum to provide a fluid brown powder. Ti content: 3.20 wt-%.

### Method two:

To tetrahydrofuran (THF for short, 45 mL) was added anhydrous MgCl₂ (1.5 g) and the resulting suspension was stirred for 2 hrs at 60 °C to get MgCl₂ dissolved totally. To the resulting solution was added silica (1.7 g) and the mixture was stirred for 1 h. Hexane (40 mL) was added and then the reaction system was cooled to room temperature under stirring. After filtration, the obtained solid was dried under vacuum to provide a composite carrier.

To a solution of TiCl₄(THF)₂ in dichloromethane (2 mL) was added a solution of electronic donor ED01 in dichloromethane (2 mL), and the resulting solution was added to the composite carrier (0.77 g) with stirring. Removing the solvent under vacuum to provide Ziegle-Natta catalyst as fluid brown powders.

### Example 33-69

The following examples are the preparation of the Ziegler-Natta catalyst containing novel electronic donor following the procedures of example **32**, and the difference and the metal content of the Ziegler-Natta catalyst are listed in Table **1**).

**Table 1**

| Example | Catalyst | Anhydrous MgCl₂ (g) | THF (mL) | TiCl₄ (mmol) | Carrier (g) | ED (mmol) | Ti (wt-%) |
|---|---|---|---|---|---|---|---|
| 33 | **SC02** | 1.0 | 40 | 3.4 | ES70(1.0) | **ED02**(3.5) | 4.61 |
| 34 | **SC03** | 1.0 | 40 | 3.4 | ES757(1.0) | **ED02**(3.5) | 4.56 |
| 35 | **SC04** | 1.0 | 40 | 3.4 | Grace955(1.0) | **ED02**(3.5) | - |
| 36 | **SC05** | 1.2 | 50 | 3.4 | ES70(1.0) | **ED05**(3.5) | 4.35 |
| 37 | **SC06** | 1.2 | 50 | 3.4 | ES70(1.0) | **ED06**(4.0) | - |
| 38 | **SC07** | 0.9 | 40 | 3.4 | ES70X(1.0) | **ED07**(6.8) | 3.75 |
| 39 | **SC08** | 1.2 | 50 | 3.4 | ES70Y(L1) | **ED08**(4.0) | - |
| 40 | **SC09** | 1.0 | 40 | 3.4 | ES70(1.0) | **ED09**(4.0) | 4.65 |
| 41 | **SC11** | 1.0 | 40 | 3.4 | ES70(1.0) | **ED13**(4.1) | 4.50 |
| 42 | **SC12** | 1.1 | 40 | 1.7 | ES70(1.3) | **ED13**(3.4) | 1.68 |
| 43 | **SC13** | 0.5 | 20 | 3.4 | ES70(1.0) | **ED13**(4.1) | 1.09 |
| 44 | **SC14** | 1.0 | 60 | 6.8 | ES70(2.0) | **ED13**(8.2) | 5.65 |
| 45 | **SC15** | 1.0 | 40 | 3.0 | ES70(1.0) | **ED13**(1.5) | 4.59 |
| 46 | **SC16** | 2.0 | 60 | 3.2 | ES757(2.0) | **ED13**(3.8) | - |
| 47 | **SC17** | 0.5 | 30 | 1.7 | ES70(0.5) | **ED14**(2.6) | - |
| 48 | **SC18** | 0.5 | 30 | 1.7 | ES70(0.5) | **ED15**(2.6) | 4.45 |
| 49 | **SC19** | 0.5 | 30 | 1.7 | ES70(0.5) | **ED16**(2.6) | - |
| 50 | **SC20** | 0.5 | 30 | 1.7 | ES70(0.5) | **ED17**(2.6) | 4.35 |
| 51 | **SC21** | 0.5 | 30 | 1.7 | ES70(0.5) | **ED18**(2.6) | 4.37 |
| 52 | **SC22** | 0.5 | 30 | 1.7 | ES70(0.5) | **ED21**(2.6) | 3.95 |
| 53 | **SC23** | 0.5 | 30 | 1.7 | ES70(0.5) | **ED22**(2.6) | - |
| 54 | **SC24** | 0.5 | 30 | 1.7 | ES70(0.5) | **ED23**(2.6) | 4.43 |
| 55 | **SC25** | 0.5 | 30 | 1.7 | ES70(0.5) | **ED24**(2.6) | - |
| 56 | **SC26** | 0.5 | 30 | 1.7 | ES70(0.5) | **ED25**(2.6) | 4.53 |
| 57 | **SC27** | 1.0 | 60 | 6.8 | ES70(2.0) | **ED26**(8.2) | - |
| 58 | **SC28** | 1.0 | 60 | 6.8 | ES757(2.0) | **ED27**(8.2) | - |
| 59 | **SC29** | 1.0 | 60 | 6.8 | ES70(2.0) | **ED2**8(8.2) | - |
| 60 | **SC30** | 1.0 | 60 | 0.8 | ES70(2.0) | **ED33**(1.8) | 0.82 |
| 61 | **SC31** | 0.5 | 60 | 0.8 | ES757(3.0) | **ED35**(1.6) | 0.40 |
| 62 | **SC32** | 1.0 | 40 | 3.4 | ES70(1.0) | **ED37**(4.1) | - |
| 63 | **SC33** | 1.0 | 40 | 3.4 | ES70(1.0) | **ED38**(4.1) | - |
| 64 | **SC34** | 1.0 | 40 | 3.4 | ES70(1.0) | **ED39**(4.1) | - |
| 65 | **SC35** | 1.0 | 40 | 3.4 | ES70(1.0) | **ED40**(4.1) | - |
| 66 | **SC36** | 1.0 | 40 | 3.4 | ES70(1.0) | **ED41**(4.1) | - |
| 67 | **SC37** | 1.0 | 40 | 3.4 | ES70(1.0) | **ED42**(4.1) | 4.10 |
| 68 | **SC38** | 1.0 | 40 | 3.4 | ES70(1.0) | **ED43**(4.1) | 3.67 |
| 69 | **SC39** | 1.0 | 40 | 3.4 | ES70(1.0) | **ED44**(4.1) | - |

### Example 70-74

The following examples are the preparation of the Ziegler-Natta catalyst containing novel electronic donor following the procedures of example **32**, and the difference and the metal content of the Ziegler-Natta catalyst are listed in Table **2**).

**Table 2**

| Example | Catalyst number | Metallic compound | Electronic donor (mmol) | Metal content (wt-%) |
|---|---|---|---|---|
| 70 | **SC40** | TiCl₄(THF)₂ | **ED13**(3.5) | 4.42 |
| 71 | **SC41** | ZrCl₄ | **ED13**(3.5) | 3.36 |
| 72 | **SC42** | CrCl₃ | **ED27**(3.5) | - |
| 73 | **SC43** | CrCl₃(THF)₃ | **ED33**(3.5) | 3.41 |
| 74 | **SC44** | VCl₃(THF)₃ | **ED35**(4.0) | - |

### Example 75-103

The following examples are ethene polymerization by slurry process.

A 500 mL stainless-steel autoclave equipped with mechanical stirrer was dried under vacuum and then purged with nitrogen three times and with ethene twice. Freshly distilled 180 g n-hexane (200 mL n-hexane + 1.0 mL AlEt₃ (3.0 M in hexane)) was transferred to the reactor and the solution was stirred (rotate speed = 150 rpm) at 60 °C. Under nitrogen atmosphere, desired amount of comonomer (in the case of the combined polymerization) and Ziegler-Natta catalyst (10 mg) were added in order then the pressure in autoclave was released. Raising the temperature of the solution to 80 °C, and then ethene gas was fed to get the pressure of autoclave to 1.0 MPa. After 5 min, the rotate speed was raised to 250 rpm and the temperature of water bath was raised to 85 °C for 1 h. The autoclave was cooled quickly to below 50 °C, and the product was dried to get polymer as particles.

The detailed experimental conditions, catalytic activity (g polymer/g catalyst), polymer molecular weight M_{w} (g/mol), polymer molecular weight distribution (PDI) and the polymer bulk density (g/cm³), etc. are listed in Table **3**. The ¹³C NMR of the ethene/1-hexene copolymer obtained in example **78** is shown in Figure **2****.**

**Table 3**

| Example | Catalyst | Comonomer | Comonomer loading (g) | Activity (g /g) | M_{w} (10⁴ g/mol) | PDI | Comonomer incorporation (mol-%) |
|---|---|---|---|---|---|---|---|
| 75 | **SC01** | - | 0 | 1000 | 11.6 | 3.46 | - |
| 76 | **SC02** | - | 0 | 930 | 12.7 | 3.35 | - |
| 77 | **SC03** | 1-hexene | 10 | 1500 | 10.8 | 3.52 | 2.05 |
| 78 | **SC04** | 1-hexene | 20 | 1200 | 10.3 | 3.56 | 1.04 |
| 79 | **SC05** | - | 0 | 850 | - | - | - |
| 80 | **SC06** | - | 0 | 1100 | - | - | - |
| 81 | **SC07** | - | 0 | 1140 | - | - | - |
| 82 | **SC08** | - | 0 | 740 | - | - | - |
| 83 | **SC09** | 1-hexene | 10 | 1360 | 10.5 | | 1.95 |
| 84 | **SC18** | - | 0 | 2000 | 16.7 | 2.45 | - |
| 85 | **SC19** | - | 0 | 900 | 18.5 | 3.25 | - |
| 86 | **SC20** | - | 0 | 3500 | 12.3 | 3.22 | - |
| 87 | **SC21** | - | 0 | 1500 | 19.4 | 3.07 | - |
| 88 | **SC22** | 1-hexene | 10 | 1840 | 15.2 | 3.20 | 2.03 |
| 89 | **SC25** | 1-hexene | 10 | 4200 | 14.5 | 3.41 | 1.68 |
| 90 | **SC26** | 1-hexene | 10 | 3700 | 13.8 | 3.35 | 1.81 |
| 91 | **SC32** | - | 0 | 1660 | 14.0 | 3.56 | - |
| 92 | **SC33** | - | 0 | 1200 | 11.2 | 1.81 | - |
| 93 | **SC34** | - | 0 | 1500 | 1.3 | 3.20 | - |
| 94 | **SC35** | - | 0 | 1430 | 1.5 | 3.11 | - |
| 95 | **SC36** | - | 0 | 1700 | 10.3 | 3.69 | - |
| 96 | **SC37** | - | 0 | 2100 | - | - | - |
| 97 | **SC38** | - | 0 | 1500 | - | - | - |
| 98 | **SC39** | - | 0 | 1250 | - | - | - |
| 99 | **SC40** | - | 0 | 1650 | - | - | - |
| 100 | **SC41** | - | 0 | 860 | - | - | - |
| 101 | **SC42** | - | 0 | 1120 | - | - | - |
| 102 | **SC43** | - | 0 | 1300 | - | - | - |
| 103 | **SC44** | - | 0 | 1000 | - | - | - |

### Example 104-121

The following examples are ethene (co)polymerization by slurry process.

A 2 L stainless-steel autoclave equipped with mechanical stirrer was dried under vacuum and then purged with nitrogen three times and with ethene twice. Freshly distilled n-hexane (400 g) was transferred to the reactor and then the solution was stirred (rotate speed = 150 rpm) at 60 °C. Under nitrogen atmosphere, Ziegler-Natta catalyst (30 mg), n-hexane (200 g), and AlEt3 (2.1 mL, 0.88 M in n-hexane solution) were added to a charging tank and were shaken sufficiently and then the charging tank were connected to the polymerization system. The solution in the charging tank was pressed into autoclave by nitrogen gas, and then the residual pressure in autoclave was released. At 70 °C, ethene gas was fed into the reactor to keep the pressure of the autoclave (the hydrogen has been pumped in first in the case of hydrogen modulation polymerization) to 0.8 MPa. After 5 min, the rotate speed was raised to 250 rpm and the temperature of water bath rose to 85 °C. in the case of combined polymerization, a certain amount of comonomers was added after the polymerization was ran for 20 min. After 2 h, the autoclave was cooled quickly to below 50 °C. The product was vented and dried to get the polymer as particles.

The detailed experimental condition, catalytic activity (g polymer/g catalyst), polymer molecular weight M_{w} (g/mol), polymer molecular weight distribution (PDI), and the polymer bulk density (g/cm³), etc. are shown in Table **4**.

| Example | Catalyst | Comonomer | Comonomer loading (g) | Activity (g/g) | M_{w} (10⁴ g/mol) | PDI | Comonomer incorporation (mol %) |
|---|---|---|---|---|---|---|---|
| 104 | **SC11** | - | 0 | 18000 | 30.2 | 2.18 | - |
| 105 | **SC11** | 1-butene | 30 | 12500 | 31.5 | 2.65 | 0.15 |
| 106 | **SC11** | 1-butene | 60 | 9500 | - | - | 0.34 |
| 107 | **SC11** | 1-hexene | 30 | 11000 | 19.8 | 3.66 | 0.61 |
| 108 | **SC11** | 1-hexane | 60 | 7400 | - | - | - |
| 109 | **SC12** | - | 0 | 5300 | - | - | - |
| 110 | **SC13** | - | 0 | 8600 | - | - | - |
| 111 | **SC15** | - | 0 | 12600 | 25.4 | 3.12 | - |
| 112 | **SC15** | 1-butene | 30 | 10500 | 19.0 | 3.45 | - |
| 113 | **SC15** | 1-hexene | 30 | 7200 | - | - | - |
| 114 | **SC15** | 1-hexane | 60 | 4700 | 17.6 | 3.19 | 0.26 |
| 115 | **SC17** | - | 0 | 16000 | - | - | - |
| 116 | **SC23** | - | 0 | 11600 | 38.4 | 2.05 | - |
| 117 | **SC24** | 1-hexane | 30 | 12200 | 20.8 | 2.46 | 0.95 |
| 118 | **SC28** | - | 0 | 3400 | - | - | - |
| 119 | **SC29** | 1-hexene | 30 | 16300 | 21.5 | 2.32 | 0.87 |
| 120 | **SC30** | - | 0 | 10400 | - | - | - |
| 121 | **SC31** | - | 0 | 12000 | - | - | - |

## Claims

1. Organic compound , **characterized in that** said compound is:

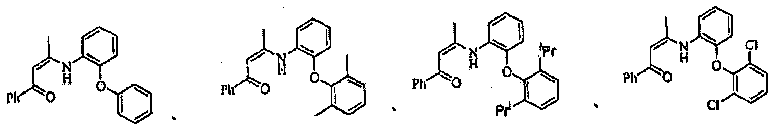

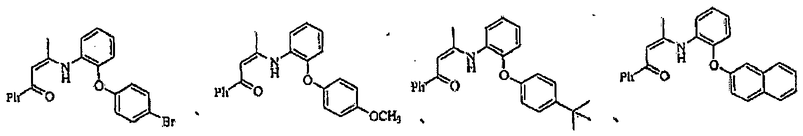

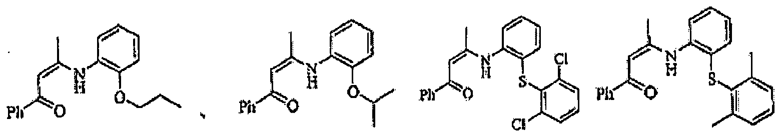

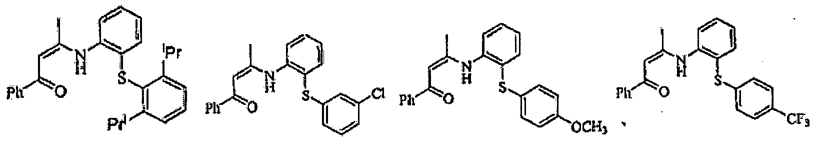

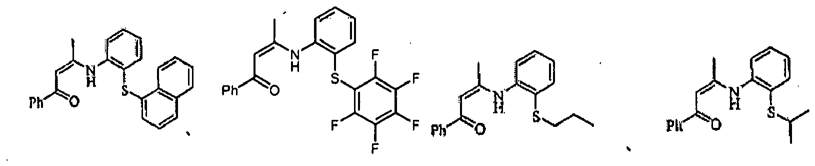

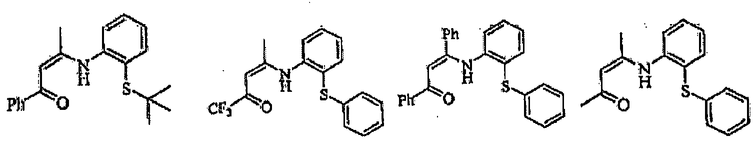

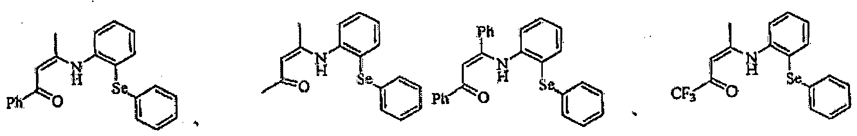

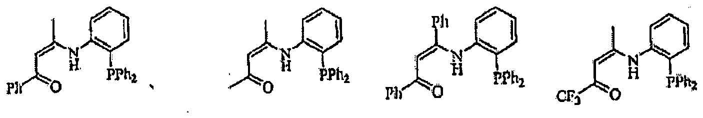

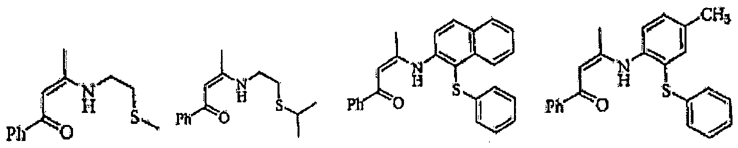

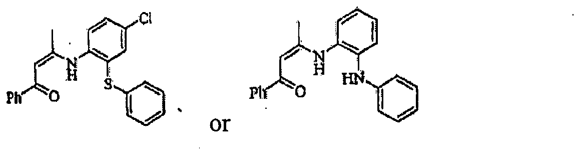

2. The organic compound containing heteroatoms of claim 1, **characterized in that** said compound is a mixture of or either one of two tautomerism in organic solvents: 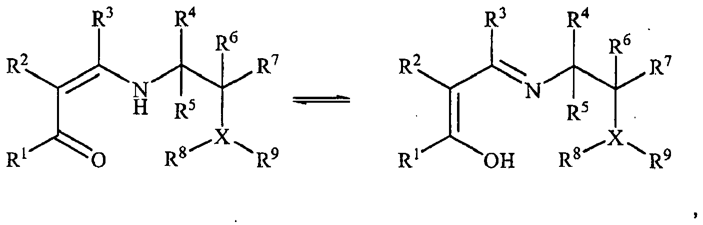 wherein the definitions of R¹-R⁹ are the same as those in claim 1.

3. The organic compound containing heteroatoms of claim 1, **characterized in that** said compound is prepared by refluxing a mixture of diketone derivative and an amine derivative in an organic solvent in presence of a correspondence catalyst in a molar ratio of (1-1.5): 1: (0.01-0.1) for 2-48 hrs,
wherein the diketone has a formula of 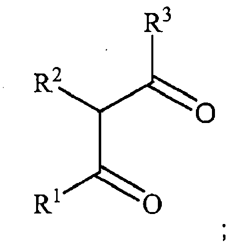 the amine has a formula of 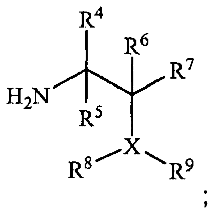 the definitions of R¹-R⁹ are the same as those in claim 1;
the catalyst is formic acid, acetic acid or TsOH.

4. A method for preparing a single-site Ziegler-Natta catalyst, **characterized in that** the organic compounds containing heteroatoms are used as electron donors, said organic compounds having a formula of 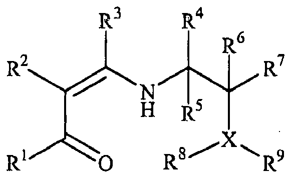 wherein:
R¹ and R² are respectively H, hydrocarbyl of C₁-C₃₀, substituted hydrocarbyl of C₁-C₃₀, aryl group of C₅-C₅₀, or substituted aryl group of C₅-C₅₀; these groups being same or different;
R³, R⁴, R⁵, R⁶ and R⁷ are respectively H, hydrocarbyl group of C₁-C₃₀, substituted hydrocarbyl group of C₁-C₃₀, aryl group of C₅-C₅₀, or substituted aryl group of C₅-C₅₀, these groups being same or different, of which, R⁴, R⁵ with R⁶ or R⁷, R⁶ with R⁸ or R⁹, R⁷ with R⁸ or R⁹ optionally form a bond or a cycle;
R⁸, and R⁹ are respectively hydrocarbyl group of C₁-C₃₀, substituted hydrocarbyl group of C₁-C₃₀, aryl group of C₅-C₅₀, or substituted aryl group of C₅-C₅₀, these groups being same or different, of which, R⁴, R⁵ with R⁶ or R⁷, R⁶ with R⁸ or R⁹, R⁷ with R⁸ or R⁹ optionally form a bond or a cycle;
X is O, S, Se or P; and
when X is O, S or Se, there is only one group, R⁸ or R⁹, on X;
the aryl group is phenyl, naphthyl, anthryl, phenanthryl or other heteroaromatic group;
the substituted hydrocarbyl group or substituted aryl group is the group substituted with hydrocarbyl, halogen, group containing silicon, group containing oxygen atom -OR¹⁰, group containing sulfur atom -SR¹¹ or -S(O)R¹², group containing nitrogen atom-NR¹³R¹⁴ or -N(O)R¹⁵R¹⁶; or group containing phosphorous atom -PR¹⁷R¹⁸ or -P(O)R¹⁹R²⁰;
R¹⁰, R¹¹, R¹², R¹³, R¹⁴, R¹⁵, R¹⁶, R¹⁷, R¹⁸, R¹⁹, or R²⁰, respectively is substituted hydrocarbyl group of C₁-C₃₀ or aryl group of C₅-C₅₀; these groups being same or different, of which, R¹³ and R¹⁴, R¹⁵ and R¹⁶, R¹⁷ and R¹⁸, R¹⁹ and R²⁰ may link to one another to form covalent bond or to form a ring.

5. The method for preparing a single-site Ziegler-Natta catalyst of claim 4, **characterized in that** the catalyst is made of magnesium compound, supporter, metal complex, and the organic compound containing heteroatom with a weight ratio of magnesium compound and supporter being 1: 0.1-20, a molar ratio of magnesium compound and metal complex being 0.5-100: 1, a molar ratio of the organic compound containing heteroatom and metal complex being 0.01-10:1, content of the metal being in a range of 0.1-15 wt%;
the magnesium compound of the single-site Ziegler-Natta catalyst is magnesium halide, alkyl magnesium, alkoxy magnesium halide, alkoxy magnesium, magnesium halide coordinate alcohol, or a mixture of two or more;
the supporter of the single-site Ziegler-Natta catalyst is an organic material or inorganic oxides containing the metal oxides of group 2, 4, 13 and 14, while the inorganic oxides is Al₂O₃, SiO₂, a mixture of oxides, clay, molecular sieve, or the oxide material provided by a gaseous metal oxide or silica compound through hydrolysis at high temperature;
the "metal complex" is represented by a formula of
MLₐ,
wherein:
a is 3 or 4;
L is a halogen atom, hydrocarbyl of C₁-C₃₀, group containing oxygen atom, or group containing nitrogen atom; each L in the formula is same or different, and may link to one another to form bonds or a ring;
the halogen atom is F, Cl, Br or I;
the group containing oxygen atom is an alkoxy-OR²³, tetrahydrofuran, or diethyl ether; the group containing nitrogen atom is - NR²⁴R²⁵;
R²³, R²⁴, R²⁵, R²⁶, and R²⁷ are respectively H, hydrocarbyl group of C₁-C₃₀ or aryl group of C₅-C₅₀, and these groups are same or different, and R²⁴ with R²⁵, R²⁶ with R²⁷ may form a bond or to a ring;
M is a group IV to group VI transition metal.

6. The method for preparing a single-site Ziegler-Natta catalyst of claim 5, **characterized in that** the Titanium compound is TiCl₄, TiCl₄(THF)₂, Ti(OCH₃)Cl₃, or Ti(OC₂H₅)Cl₃, and THF being tetrahydrofuran.

7. The method for preparing a single-site Ziegler-Natta catalyst of claim 5, **characterized in that** the metal complex is TiCl₄, TiCl₄(THF)₂, Ti(NMe₂)₄, Ti(CH₂Ph)₄, ZrCl₄, Zr(NMe₂)₄, CrCl₃, CrCl₃(THF)₃, VCl₃, or VCl₃(THF)₃.

8. The method for preparing a single-site Ziegler-Natta catalyst of claim 5, **characterized in that**: the supporter is SiO₂.

9. The method for preparing a single-site Ziegler-Natta catalyst of claim 5, **characterized in that** the organic compound containing heteroatom is used as an electronic donor to prepare the single site Ziegle-Natta catalyst in preparation steps of:
(1) treating an organic or inorganic solid or a mixture thereof at 30-1000°C for 1-24 hrs under an inert or reduced atmosphere;
(2) at room temperature to 70°C, dissolving a magnesium compound in an THF to form a solution with a ratio of magnesium compound and THF at 1 g: 10-100 mL;
(3) adding to the solution (2) the supporter obtained in (1), a metal complex, and electron donor sequentially, to form a reaction mixture, and keeping the reaction mixture at room temperature to 100°C for 2-48 hrs, while weight ratio of the magnesium compound and the supporter is 1: 0.1-20; the molar ratio of the magnesium compound and the metal complex being 0.5-100: 1; molar ratio of the organic compound containing heteroatom and the metal complex being 0.01-10:1.

10. The method for preparing a single-site Ziegler-Natta catalyst of claim 9, **characterized in that** the magnesium compound reacts with the metal complex for 2-48 hrs at room temperature to 100 °C before being treated with a pretreated carrier, then, a solid obtained therefrom is treated with the electronic donor for 2-48 hrs at room temperature to 100 °C to provide the Ziegler-Natta catalyst.

11. The method for preparing a single-site Ziegler-Natta catalyst of claim 9, **characterized in that** the magnesium compound is treated with a carrier for 2-48 h at room temperature to 100 °C to get a composite carrier, and then reacts with a solution of the electron donor and the metal complex for 2-48 hrs at room temperature to 100 °C to provide the Ziegler-Natta catalyst.

12. The single-site Ziegler-Natta catalyst as prepared by the method of claim 5, **characterized in that** the catalyst is suitable for ethylene polymerization, ethylene/α-olefin copolymerization and ethylene/cycloolefin copolymerization.

13. The single site Ziegler-Natta catalyst of claim 12, **characterized in that** an alkyl aluminium, alkyl aluminoxane, or a mixture of two or more is used as a cocatalyst in the polymerization, the cocatalyst being AlEt₃, Al(i-Bu)₃, AlEt₂Cl, Al(n-Hex)₃, MAO, EAO, MMAO or a mixture of two or more; molar ratio of Al/Ti is 20-1000; α-olefins are the olefins of C₃-C₂₀; cycloolefins are cyclopetene, cyclohexene, norbornene, or their derivatives, and all the α-olefins and cycloolefins optionally contain hydroxyl group, carboxyl group, ester group, or amine group.

14. The single site Ziegler-Natta catalyst of claim 12, **characterized in that** the polymerization is run in a slurry process or gas process.

15. The single site Ziegler-Natta catalyst of claim 14, **characterized in that** in the slurry polymerization process, the polymerization is generally conducted under 0.1-10 MPa of total pressure with hydrogen pressure being 0-1.0 MPa, temperature being 80-120 °C, the polymerization is conducted under supercritical state or subcritical state in propane, isobutene or hexane; in the gas polymerization process, the polymerization is conducted under 1.0-10.0 Mpa at 40-100 °C in gas fluidized bed or gas autoclave.

## Patentansprüche

1. Organische Verbindung, **dadurch gekennzeichnet, dass** die Verbindung die folgende ist:

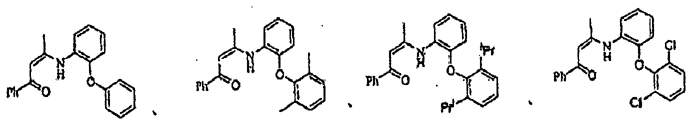

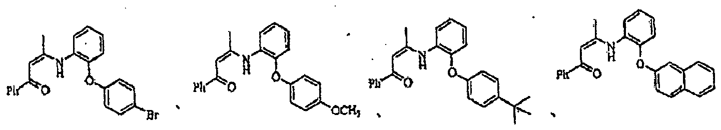

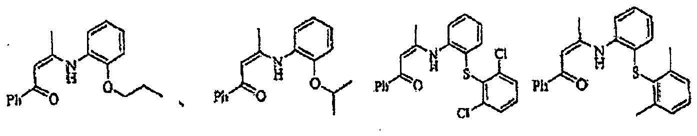

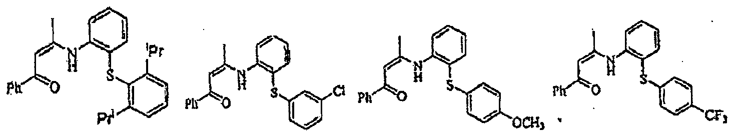

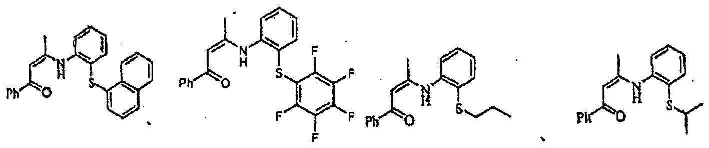

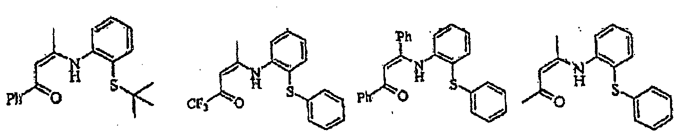

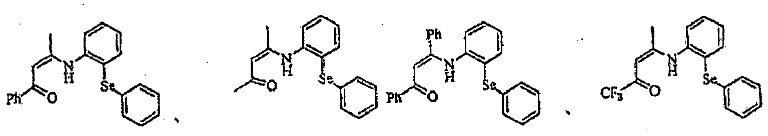

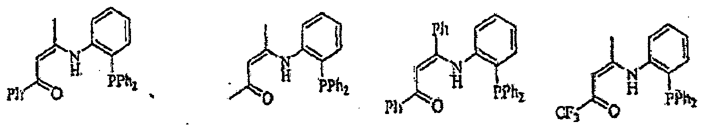

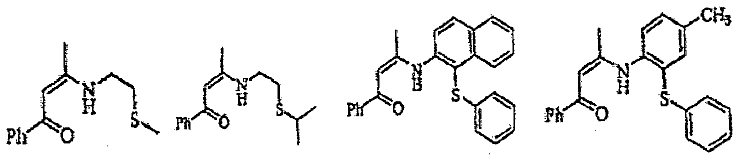

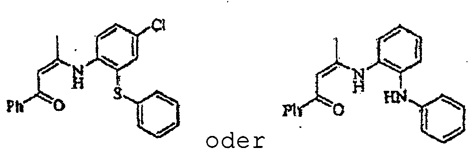

2. Organische Verbindung, die Heteroatome nach Anspruch 1 enthält, **dadurch gekennzeichnet, dass** die Verbindung eine Mischung von zwei oder von einer von zwei Tautomerien in organischen Lösemitteln ist: 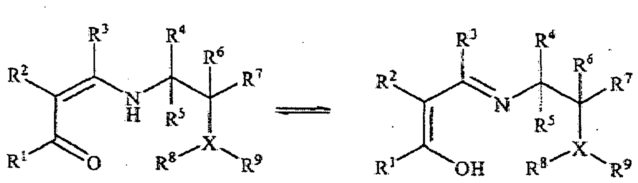 wobei die Definitionen von R¹ bis R⁹ dieselben sind wie diejenigen in Anspruch 1.

3. Organische Verbindung, die Heteroatome nach Anspruch 1 enthält, **dadurch gekennzeichnet, dass** die Verbindung hergestellt wird durch ein Rückfließen einer Mischung von Diketonderivat und eines Aminderivats in einem organischen Lösemittel in Gegenwart eines entsprechenden Katalysators in einem molaren Verhältnis von (1-1,5) : 1 : (0,01-0,1) während einer Zeitdauer von 2 bis 48 Stunden,
wobei das Diketon eine Formel aufweist von: 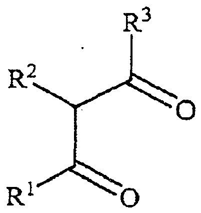 wobei das Amin eine Formel aufweist von: 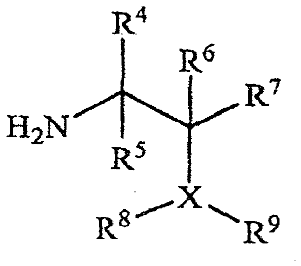 wobei die Definitionen von R¹ bis R⁹ dieselben sind wie diejenigen in Anspruch 1; der Katalysator ist Ameisensäure, Essigsäure oder TsOH.

4. Verfahren zum Herstellen eines Ziegler-Natta-Katalysators mit einer (einzigen) aktiven Stelle (Single-Site Katalysator) **dadurch gekennzeichnet, dass** die organischen Verbindungen, die Heteroatome enthalten, als Elektronendonoren verwendet werden, wobei die organischen Verbindungen eine Formel aufweisen von: 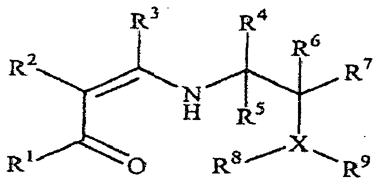 wobei:
R¹ und R² jeweils H, ein Hydrocarbyl von C₁-C₃₀, substituiertes Hydrocarbyl von C₁-C₃₀, eine Arylgruppe von C₅-C₅₀ oder eine substituierte Arylgruppe von C₅-C₅₀ darstellen, wobei diese Gruppen dieselben oder verschieden sind;
R³, R⁴, R⁵ und R⁷ jeweils H, eine Hydrocarbylgruppe von C₁-C₃₀, eine substituierte Hydrocarbylgruppe von C₁-C₃₀, eine Arylgruppe von C₅-C₅₀ oder eine substituierte Arylgruppe von C₅-C₅₀ darstellen, wobei diese Gruppen dieselben oder verschieden sind, von denen R⁴, R⁵ mit R⁶ oder R⁷, R⁶ mit R⁸ oder R⁹, R⁷ mit R⁸ oder R⁹ wahlweise eine Bindung oder einen Ring bilden;
R⁸ und R⁹ jeweils eine Hydrocarbylgruppe von C₁-C₃₀, eine substituierte Hydrocarbylgruppe von C₁-C₃₀, eine Arylgruppe von C₅-C₅₀ oder eine substituierte Arylgruppe von C₅-C₅₀ darstellen, wobei diese Gruppen dieselben oder verschieden sind, von denen R⁴, R⁵ mit R⁶ oder R⁷, R⁶ mit R⁸ oder R⁹, R⁷ mit R⁸ oder R⁹ wahlweise eine Bindung oder einen Ring bilden;
X für O, S, Se oder P steht; und
wenn X für O, S oder Se steht, dann gibt es nur eine Gruppe, R⁸ oder R⁹, auf X;
die Arylgruppe Phenyl, Naphthyl, Anthryl, Phenanthryl oder eine andere heteroaromatische Gruppe ist;
die substituierte Hydrocarbylgruppe oder substituierte Arylgruppe für die Gruppe, die mit einem Hydrocarbylkohlenstoff, Halogen substituiert ist, für die Gruppe, die Silizium enthält, für die Gruppe, die ein Sauerstoffatom -OR¹⁰ enthält, für die Gruppe, die ein Schwefelatom -SR¹¹ oder -S(O)R¹² enthält, für die Gruppe, die ein Stickstoffatom -NR¹³R¹⁴ oder -N(O)R¹⁵R¹⁶ enthält, oder für die Gruppe steht, die ein Phosphoratom -PR¹⁷R¹⁸ oder -P(O)R¹⁹R²⁰ enthält;
R¹⁰, R¹¹, R¹², R¹³, R¹⁴, R¹⁵, R¹⁶, R¹⁷, R¹⁸, R¹⁹ oder R²⁰ jeweils mit einer Hydrocarbylgruppe von C₁-C₃₀ oder einer Arylgruppe von C₅-C₅₀ substituiert ist, wobei diese Gruppen dieselben oder verschieden sind, von denen sich R¹³ und R¹⁴, R¹⁵ und R¹⁶, R¹⁷ und R¹⁸, R¹⁹ und R²⁰ miteinander verbinden, um eine kovalente Bindung oder einen Ring zu bilden

5. Verfahren zum Herstellen eines Ziegler-Natta-Katalysators mit einer aktiven Stelle nach Anspruch 4, **dadurch gekennzeichnet, dass** der Katalysator aus einer Magnesiumverbindung, einem Träger, einem Metallkomplex und der organischen Verbindung, die Heteroatome enthält, mit einem Gewichtsverhältnis der Magnesiumverbindung und des Trägers, das 1 : 0,1-20 beträgt, mit einem molaren Verhältnis von Magnesiumverbindung und Metallkomplex, das 0,5-100 : 1 beträgt, mit einem molaren Verhältnis der organischen Verbindung, die Heteroatome enthält, und des Metallkomplexes, das 0,01-10 : 1 beträgt, hergestellt ist, wobei der Metallgehalt in einem Bereich von 0,1-15 Gew.% liegt;
die Magnesiumverbindung des Ziegler-Natta-Katalysators mit einer aktiven Stelle Magnesiumhalid, Alkylmagnesium, Alkoxymagnesiumhalid, Alkoxymagnesium, koordinativer Magnesiumhalidalkohol oder eine Mischung von zwei oder mehreren davon ist;
der Träger des Ziegler-Natta-Katalysators mit einer aktiven Stelle ein organisches Material ist oder aus anorganischen Oxiden besteht, die die Metalloxide der Gruppe 2, 4, 13 und 14 enthalten, während die anorganischen Oxide Al₂O₃, SiO₂, eine Mischung von Oxiden, Ton, ein Molekularsieb oder das Oxidmaterial, das durch ein gasförmiges Metalloxid oder durch Siliciumdioxid durch Hydrolyse bei hohen Temperaturen bereitgestellt wird, sind;
der "Metallkomplex" durch folgende Formel dargestellt wird:
MLa,
wobei:
a für 3 oder 4 steht;
L ein Halogenatom, ein Hydrocarbyl von C₁-C₃₀, eine Gruppe, die ein Sauerstoffatom enthält, oder eine Gruppe, die ein Stickstoffatom enthält, ist; jedes L in der Formel ist gleich oder verschieden, und die L's können sich miteinander verbinden, um Bindungen oder einen Ring zu bilden;
das Halogenatom F, Cl, B oder I ist;
die Gruppe, die ein Sauerstoffatom enthält, ein Alkoxy-OR²³, Tetrahydrofuran oder ein Diethylether ist; die Gruppe, die ein Stickstoffatom enthält,-NR²⁴R²⁵ ist;
R²³, R²⁴, R²⁵, R²⁶ und R²⁷ jeweils H, eine Hydrocarbylgruppe von C₁-C₃₀ oder eine Arylgruppe von C₅-C₅₀ sind, und diese Gruppen sind gleich oder verschieden, und R²⁴ kann mit R²⁵, R²⁶ kann mit R²⁷ eine Bindung oder einen Ring bilden;
M ist ein Übergangsmetall von der Gruppe IV zu der Gruppe VI.

6. Verfahren zum Herstellen eines Ziegler-Natta-Katalysators mit einer aktiven Stelle nach Anspruch 5, **dadurch gekennzeichnet, dass** die Titanverbindung TiCl₄, TiCl₄ (THF)₂, Ti(OCH₃)Cl₃ oder Ti(OC₂H₅)Cl₃ und THF, das Tetrahydrofuran ist, ist.

7. Verfahren zum Herstellen eines Ziegler-Natta-Katalysators mit einer aktiven Stelle nach Anspruch 5, **dadurch gekennzeichnet, dass** der Metallkomplex TiCl₄, TiCl₄(THF)₂, Ti(NMe₂)₄, Ti(CH₂PH)₄, ZrCl₄, Zr(NMe₂)₄, CrCl₃, CrCl₃(THF)₃, VCl₃ oder VCl₃(THE)₃ ist.

8. Verfahren zum Herstellen eines Ziegler-Natta-Katalysators mit einer aktiven Stelle nach Anspruch 5, **dadurch gekennzeichnet, dass**: der Träger SiO₂ ist.

9. Verfahren zum Herstellen eines Ziegler-Natta-Katalysators mit einer aktiven Stelle nach Anspruch 5, **dadurch gekennzeichnet, dass** die organische Verbindung, die Heteroatome enthält, als ein Elektronendonor verwendet wird, um den Ziegler-Natta-Katalysator mit einer aktiven Stelle mit den folgenden Herstellungsschritten herzustellen:
(1) Behandeln eines organischen oder anorganischen Feststoffs oder eine Mischung davon bei 30 bis 1000 °C während einer Zeitdauer von 1 bis 24 Stunden unter einer inerten oder reduzierten Atmosphäre;
(2) bei Raumtemperatur bis hin zu 70 °C Auflösen einer Magnesiumverbindung in THF, um eine Lösung mit einem Verhältnis der Magnesiumverbindung und des THF von 1 g : 10-100 ml zu bilden;
(3) nacheinander Hinzugeben des in (1) erhaltenen Trägers, eines Metallkomplexes und eines Elektronendonors zu der Lösung (2), um eine Reaktionsmischung zu bilden, und Halten der Reaktionsmischung bei Raumtemperatur bis hin zu 100 °C während einer Zeitdauer von 2 bis 48 Stunden, während das Gewichtsverhältnis der Magnesiumverbindung und des Trägers 1 : 0,1-20 beträgt; das molare Verhältnis der Magnesiumverbindung und des Metallkomplexes beträgt 0,5-100 : 1; das molare Verhältnis der organischen Verbindung, die Heteroatome enthält, und des Metallkomplexes beträgt 0,01-10 : 1.

10. Verfahren zum Herstellen eines Ziegler-Natta-Katalysators mit einer aktiven Stelle nach Anspruch 9, **dadurch gekennzeichnet, dass** die Magnesiumverbindung mit dem Metallkomplex bei Raumtemperatur bis hin zu 100 °C während einer Zeitdauer von 2 bis 48 Stunden reagiert, bevor sie mit einem vorbehandelten Träger behandelt wird, dann wird ein davon erhaltener Feststoff mit dem Elektronendonor bei Raumtemperatur bis hin zu 100 °C während einer Zeitdauer von 2 bis 48 Stunden behandelt, um den Ziegler-Natta-Katalysator mit einer aktiven Stelle bereitzustellen.

11. Verfahren zum Herstellen eines Ziegler-Natta-Katalysators mit einer aktiven Stelle nach Anspruch 9, **dadurch gekennzeichnet, dass** die Magnesiumverbindung bei Raumtemperatur bis hin zu 100°C während einer Zeitdauer von 2 bis 48 Stunden mit einem Träger behandelt wird, um eine Trägerzusammensetzung zu erhalten, und dass sie dann mit einer Lösung des Elektronendonors und des Metallkomplexes bei Raumtemperatur bis hin zu 100 °C während einer Zeitdauer von 2 bis 48 Stunden reagiert, um den Ziegler-Natta-Katalysator mit einer aktiven Stelle bereitzustellen.

12. Ziegler-Natta-Katalysator mit einer aktiven Stelle, wie er durch das Verfahren nach Anspruch 5 hergestellt worden ist, **dadurch gekennzeichnet, dass** der Katalysator für eine Ethylenpolymerisation, Ethylen/α-Olefin-Copolymerisation und für eine Ethylen/Cycloolefin-Copolymerisation geeignet ist.

13. Ziegler-Natta-Katalysator mit einer aktiven Stelle nach Anspruch 12, **dadurch gekennzeichnet, dass** ein Alkyaluminium, Alkyaluminoxan oder eine Mischung von zwei oder mehreren als Cokatalysator in der Polymerisation verwendet wird, wobei der Cokatalysator AlEt₃, Al(i-Bu)₃, AlEt₂Cl, Al(n-Hex)₃, MAO, EAO, MMAO oder eine Mischung von zwei oder mehreren ist; das molare Verhältnis von Al/Ti beträgt 20 bis 1000; α-Olefine sind die Olefine von C₃-C₂₀; Cycloolefine sind Cyclopeten, Cylohexen, Norbornen oder ihre Derivative, und alle α-Olefine und Cycloolefine enthalten wahlweise eine Hydroxylgruppe, Carboxylgruppe, Estergruppe oder eine Amingruppe.

14. Ziegler-Natta-Katalysator mit einer aktiven Stelle nach Anspruch 12, **dadurch gekennzeichnet, dass** die Polymerisation in einem aufgeschlämmten Verfahren (Slurry-Verfahren) oder in einem Gasverfahren abläuft.

15. Ziegler-Natta-Katalysator mit einer aktiven Stelle nach Anspruch 12, **dadurch gekennzeichnet, dass** in dem Verfahren der Aufschlämmungspolymerisation die Polymerisation im Allgemeinen unter 0,1 bis 10 MPa Gesamtdruck mit einem Wasserstoffdruck, der bei 0,1 bis 1,0 MPa liegt, geführt wird, wobei die Temperatur bei 80 bis 120 °C liegt; die Polymerisation wird unter einem superkritischen Zustand oder unterkritischen Zustand in Propan, Isobuten oder Hexan ausgeführt; in dem Verfahren der Gaspolymerisation wird die Polymerisation unter 1,0 bis 10,0 MPa bei 40 bis 100 °C in einem Gasfließbett oder in einem Gasautoklav durchgeführt.

## Revendications

1. Composé organique, **caractérisé en ce que** ledit composé est :

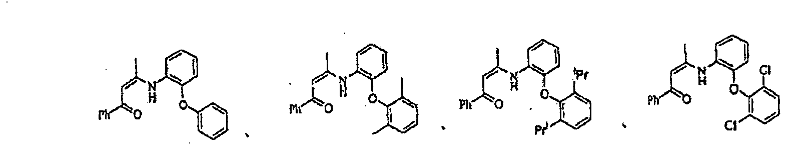

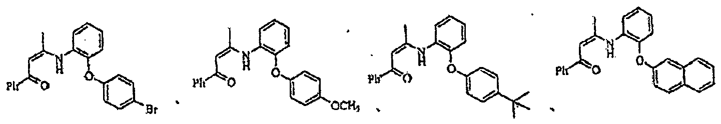

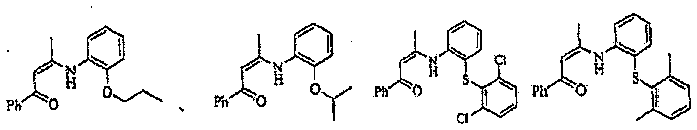

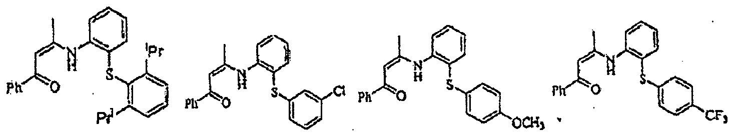

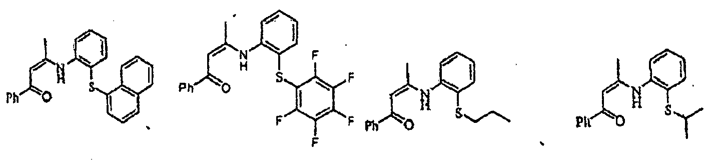

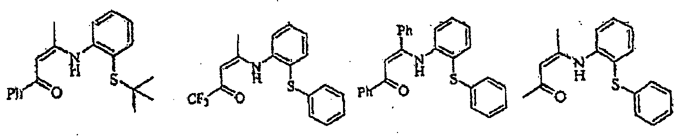

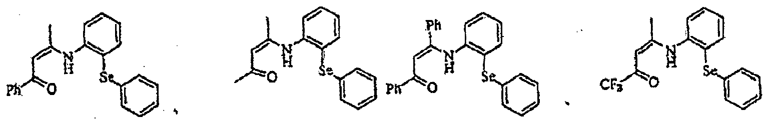

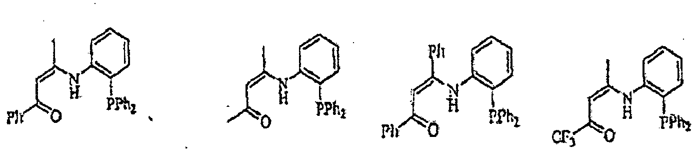

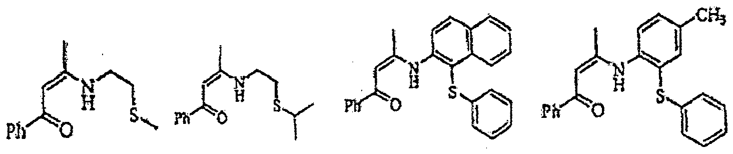

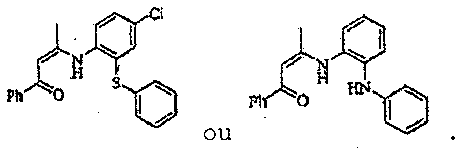

2. Composé organique contenant des hétéroatomes selon la revendication 1, **caractérisé en ce que** ledit composé est un mélange de ou l'une quelconque parmi deux tautoméries dans des solvants organiques : 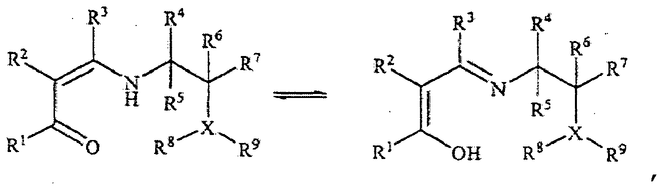 dans lesquels les définitions de R¹ - R⁹ sont identiques à celles selon la revendication 1.

3. Composé organique contenant des hétéroatomes selon la revendication 1, **caractérisé en ce que** ledit composé est préparé en mettant au reflux un mélange d'un dérivé de dicétone et d'un dérivé d'amine dans un solvant organique en présence d'un catalyseur de correspondance dans un rapport molaire de (1 - 1,5) : 1 : (0,01 - 0,1) pendant 2 à 48 heures,
dans lequel la dicétone a la formule de 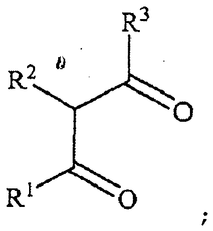 l'amine a la formule de 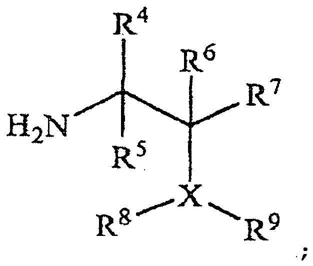 les définitions de R¹ - R⁹ sont identiques à celles selon la revendication 1 ;
le catalyseur est de l'acide formique, de l'acide acétique ou TsOH.

4. Procédé de préparation d'un catalyseur du type Ziegler - Natta à un seul site, **caractérisé en ce que** les composés organiques contenant des hétéroatomes sont utilisés comme des donneurs d'électrons, lesdits composés organiques ayant une formule de 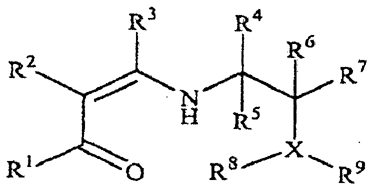 dans laquelle :
R¹ et R² sont respectivement H, un hydrocarbyle de C₁ - C₃₀, un hydrocarbyle substitué de C₁ - C₃₀, un groupe aryle de C₅ - C₅₀, ou un groupe aryle substitué de C₅ - C₅₀, ces groupes étant identiques ou différents ;
R³, R⁴, R⁵, R⁶ et R⁷ sont respectivement H, un groupe hydrocarbyle de C₁ - C₃₀, un groupe hydrocarbyle substitué de C₁ - C₃₀, un groupe aryle de C₅ - C₅₀, ou un groupe aryle substitué de C₅ - C₅₀, ces groupes étant identiques ou différents, parmi lesquels R⁴, R⁵ avec R⁶ ou R⁷, R⁶ avec R⁸ ou R⁹, R⁷ avec R⁸ ou R⁹ forment éventuellement une liaison ou un cycle ;
R⁸ et R⁹ sont respectivement un groupe hydrocarbyle de C₁ - C₃₀, un groupe hydrocarbyle substitué de C₁ - C₃₀, un groupe aryle de C₅ - C₅₀, ou un groupe aryle substitué de C₅ - C₅₀, ces groupes étant identiques ou différents, parmi lesquels R⁴, R⁵ avec R⁶ ou R⁷, R⁶ avec R⁸ ou R⁹, R⁷ avec R⁸ ou R⁹ forment éventuellement une liaison ou un cycle ;
X est O, S, Se ou P ; et
quand X est O, S ou Se, il n'y a qu'un groupe, R⁸ ou R⁹, sur X ;
le groupe aryle est phényle, naphtyle, anthryle, phénanthryle ou un autre groupe hétéroaromatique ;
le groupe hydrocarbyle substitué ou le groupe aryle substitué est le groupe substitué avec hydrocarbyle, halogène, un groupe contenant du silicium, un groupe contenant un atome d'oxygène -OR¹⁰, un groupe contenant un atome de soufre -SR¹¹ ou -S(O)R¹², un groupe contenant un atome d'azote -NR¹³R¹⁴ ou -N(O)R¹⁵R¹⁶, ou un groupe contenant un atome de phosphore -PR¹⁷R¹⁸ ou -P(O)R¹⁹R²⁰;
R¹⁰, R¹¹, R¹², R¹³, R¹⁴, R¹⁵, R¹⁶, R¹⁷, R¹⁸, R¹⁹, ou R²⁰, respectivement est un groupe hydrocarbyle substitué de C₁ - C₃₀ ou un groupe aryle de C₅ - C₅₀, ces groupes étant identiques ou différents, parmi lesquels R¹³ et R¹⁴, R¹⁵ et R¹⁶, R¹⁷ et R¹⁸, R¹⁹ et R²⁰ peuvent se lier l'un à l'autre pour former une liaison covalente ou pour former un noyau.

5. Procédé de préparation d'un catalyseur du type Ziegler - Natta à un seul site selon la revendication 4, **caractérisé en ce que** le catalyseur est fabriqué d'un composé de magnésium, d'un supporter et d'un complexe métallique, et le composé organique contenant un hétéroatome avec un rapport pondéral du composé de magnésium et du supporter étant de 1 : 0,1 - 20, un rapport molaire du composé de magnésium et du complexe métallique étant de 0,5 - 100 : 1 , un rapport molaire du composé organique contenant un hétéroatome et le complexe métallique étant de 0,01 - 10 : 1, la teneur en métal étant dans une gamme de 0,1 - 15 % en poids ;
le composé de magnésium du catalyseur du type Ziegler - Natta à un seul site est un halogénure de magnésium, un magnésium d'alkyle, un halogénure de magnésium d'alkoxy, du magnésium d'alkoxy, de l'alcool coordonné d'halogénure de magnésium, ou un mélange de deux ou plus ;
le supporter du catalyseur du type Ziegler - Natta à un seul site est un matériau organique ou des oxydes minéraux contenant les oxydes métalliques du groupe 2, 4, 13 et 14, tandis que les oxydes minéraux sont Al₂O₃, SiO₂, un mélange d'oxydes, de l'argile, un tamis moléculaire, ou le matériau d'oxyde fourni par un oxyde de métal gazeux ou un composé de silice au moyen d'une hydrolyse à haute température ;
le « complexe métallique » est représenté par une formule de
MLa,
dans laquelle :
a est 3 ou 4 ;
L est un atome d'halogène, un hydrocarbyle de C₁ - C₃₀, un groupe contenant un atome d'oxygène, ou un groupe contenant un atome d'azote ; chaque L dans la formule est identique ou différent, et peuvent se lier l'un à l'autre pour former des liaisons ou un noyau ;
l'atome d'halogène est F, Cl, Br ou I ;
le groupe contenant un atome d'oxygène est un alkoxy-OR²³, du tétrahydrofurane, ou de l'éther diéthylique ; le groupe contenant l'atome d'azote est -NR²⁴R²⁵ ;
R²³, R²⁹, R²⁵, R²⁶, et R²⁷ sont respectivement H, un groupe hydrocarbyle de C₁ - C₃₀ ou un groupe aryle de C₅ - C₅₀, et ces groupes sont identiques ou différents, et R²⁴ avec R²⁵, R²⁶ avec R²⁷ peuvent former une liaison ou un noyau ;
M est un métal de transition du groupe IV au groupe VI.

6. Procédé de préparation d'un catalyseur du type Ziegler - Natta à un seul site selon la revendication 5, **caractérisé en ce que** le composé de titane est TiCl₄, TiCl₄(THF)₂, Ti(OCH₃)Cl₃, ou Ti(OC₂H₅)Cl₃, et THF étant du tétrahydrofurane.

7. Procédé de préparation d'un catalyseur du type Ziegler - Natta à un seul site selon la revendication 5, **caractérisé en ce que** le complexe métallique est TiCl₄, TiCl₄(THF)₂, Ti(NMe₂)₄, Ti(CH₂Ph)₄, ZrCl₄, Zr(NMe₂)₄, CrCl₃, CrCl₃(THF)₃, VCl₃, ou VCl₃(THF)₃.

8. Procédé de préparation d'un catalyseur du type Ziegler - Natta à un seul site selon la revendication 5, **caractérisé en ce que** : le supporter est SiO₂.

9. Procédé de préparation d'un catalyseur du type Ziegler - Natta à un seul site selon la revendication 5, **caractérisé en ce que** le composé organique contenant un hétéroatome est utilisé comme un donneur électronique pour préparer le catalyseur du type Ziegler - Natta à un seul site dans les étapes de préparation consistant à :
(1) traiter un solide organique ou minéral ou un mélange de ceux-ci à 30 - 1000° C pendant 1 à 24 h sous une atmosphère inerte ou réduite ;
(2) à une température ambiante jusqu'à 70° C, dissoudre un composé de magnésium dans un THF pour former une solution avec un rapport du composé de magnésium et du THF à 1 g : 10 - 100 ml ;
(3) ajouter à la solution (2) le supporter obtenu en (1), un complexe métallique, et un donneur d'électrons de façon séquentielle, pour former un mélange réactionnel, et conserver le mélange réactionnel à la température ambiante à 100° C pendant 2 - 48 heures, tandis que le rapport pondéral du composé de magnésium et du supporter est 1 : 0,1 - 20 ; le rapport molaire du composé de magnésium et du complexe métallique étant de 0,5 - 100 : 1 ; le rapport molaire du composé organique contenant un hétéroatome et du complexe métallique étant de 0,01 - 10 : 1.

10. Procédé de préparation d'un catalyseur du type Ziegler - Natta à un seul site selon la revendication 9, **caractérisé en ce que** le composé de magnésium réagit avec le complexe métallique pendant 2 à 48 h à une température ambiante jusqu'à 100° C avant d'être traité avec un support prétraité, puis, un solide obtenu de celui-ci est traité avec le donneur électronique pendant 2 à 48 h à une température ambiante jusqu'à 100° C pour fournir un catalyseur du type Ziegler - Natta.

11. Procédé de préparation d'un catalyseur du type Ziegler - Natta à un seul site selon la revendication 9, **caractérisé en ce que** le composé de magnésium est traité avec un support pendant 2 à 48 h à une température ambiante jusqu'à 100° C pour obtenir un support composite, puis réagit avec une solution du donneur d'électrons et du complexe métallique pendant 2 à 48 h à une température ambiante jusqu'à 100° C pour fournir le catalyseur du type Ziegler - Natta.

12. Catalyseur du type Ziegler - Natta à un seul site tel que préparé par le procédé selon la revendication 5, **caractérisé en ce que** le catalyseur est approprié pour une polymérisation éthylénique, une copolymérisation éthylénique/α-oléfinique et une copolymérisation éthylénique/cyclo-oléfinique.

13. Catalyseur du type Ziegler - Natta à un seul site selon la revendication 12, **caractérisé en ce qu'**un aluminium d'alkyle, un aluminoxane d'alkyle, ou un mélange de deux ou plusieurs est utilisé comme un co-catalyseur dans la polymérisation, le co-catalyseur étant AlEt₃, Al(i-Bu)₃, AlEt₂Cl, Al(n-Hex)₃, MAO, EAO, MMAO ou un mélange de deux ou plusieurs ; le rapport molaire d'Al/Ti est de 20 - 1000 ; les α-oléfines sont les oléfines de C₃ - C₂₀ ; les cyclo-oléfines sont du cyclopentène, du cyclohéxène, du norbornène, ou leurs dérivés, et toutes les α-oléfines et les cyclo-oléfines contiennent éventuellement un groupe hydroxyle, un groupe carboxyle, un groupe ester, ou un groupe amine.

14. Catalyseur du type Ziegler - Natta à un seul site selon la revendication 12, caractérisé en ce la polymérisation est effectuée dans un processus boueux ou un processus gazeux.

15. Catalyseur du type Ziegler - Natta à un seul site selon la revendication 14, **caractérisé en ce que** dans le processus boueux de polymérisation, la polymérisation est généralement menée sous une pression totale de 0,1 - 10 MPa avec une pression d'hydrogène étant de 0 - 1,0 MPa, la température étant de 80 - 120° C ; la polymérisation est menée sous un état supercritique ou un état sous-critique dans du propane, de l'isobutène ou de l'hexane ; dans le processus gazeux de polymérisation, la polymérisation est menée sous une pression de 1,0 - 10,0 MPa à 40 - 100° C dans un lit fluidisé de gaz ou un autoclave à gaz.
